(19) Europäisches Patentamt
European Patent Office
Office européen des brevets

(11) Veröffentlichungsnummer: **0 589 318 B1**

# (12) EUROPÄISCHE PATENTSCHRIFT

(45) Veröffentlichungstag der Patentschrift: **15.11.95**

(51) Int. Cl.[6]: **C07D 491/04**, A61K 31/435, C07D 519/00

(21) Anmeldenummer: **93114666.6**

(22) Anmeldetag: **13.09.93**

## (54) Antibakterielle 7-(Aminomethyl-oxa-7-aza-bicyclo 3.3.0 oct-7-yl)chinolon- und -naphthyridoncarbonsäure-Derivate.

(30) Priorität: **25.09.92 DE 4232172**

(43) Veröffentlichungstag der Anmeldung:
**30.03.94 Patentblatt 94/13**

(45) Bekanntmachung des Hinweises auf die Patenterteilung:
**15.11.95 Patentblatt 95/46**

(84) Benannte Vertragsstaaten:
**AT BE CH DE DK ES FR GB GR IE IT LI LU MC NL PT SE**

(56) Entgegenhaltungen:
**EP-A- 0 350 733**
**WO-A-92/10191**

**PATENT ABSTRACTS OF JAPAN vol. 15, no. 440 (C-0883)11. November 1991 & JP-A-03 188 080 (BANYU PHARMACEUTICAL CO., LTD.) 16. August 1991**

(73) Patentinhaber: **BAYER AG**

**D-51368 Leverkusen (DE)**

(72) Erfinder: **Petersen, Uwe, Dr.**
**Auf dem Forst 4**
**D-51375 Leverkusen (DE)**
Erfinder: **Schenke, Thomas, Dr.**
**Mühlenstrasse 113**
**D-51469 Bergisch Gladbach (DE)**
Erfinder: **Grohe, Klaus, Dr.**
**Am Wasserturm 10**
**D-51519 Odenthal (DE)**
Erfinder: **Bremm, Klaus-Dieter, Dr.**
**Giradetstrasse 120**
**D-42109 Wuppertal (DE)**
Erfinder: **Endermann, Rainer, Dr.**
**In den Birken 152a**
**D-42113 Wuppertal (DE)**
Erfinder: **Metzger, Karl Georg, Dr.**
**Pahlkestrasse 75**
**D-42115 Wuppertal (DE)**

EP 0 589 318 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Chinolon- und -naphthyridoncarbonsäure-Derviate, die in 7-Stellung durch einen 1- (oder 5-)Aminomethyl-2- (oder 3-)oxa-7-aza-bicyclo[3.3.0]oct-7-yl-Rest substituiert sind, ihre Salze, Verfahren zu ihrer Herstellung sowie diese enthaltende antibakterielle Mittel.

Es sind bereits aus der europäischen Patentanmeldung 350 733 sowie der japanischen Patentanmeldung 3 188 080 Chinoloncarbonsäuren bekannt geworden, die in 7-Stellung durch einen Morpholinopyrrolidinyl-Rest substituiert sind.

Es wurde jetzt gefunden, daß die Verbindungen der Formel (I)

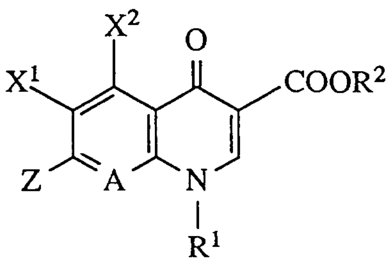

(I),

in welcher

- $X^1$ für Halogen oder Nitro,
- $X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Methyl,
- $R^1$ für Alkyl mit 1 bis 4 Kohlenstoffatomen, Alkenyl mit 2 bis 4 Kohlenstoffatomen, gegebenenfalls durch Halogen substituiertes Cycloalkyl mit 3 bis 6 Kohlenstoffatomen, Bicyclo[1.1.1]pent-1-yl, 1,1-Dimethylpropargyl, 3-Oxetanyl, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
- $R^2$ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl,
- Z für einen Rest der Struktur

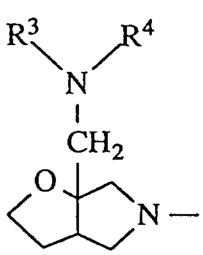 , 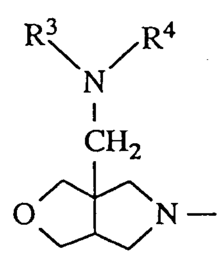 oder 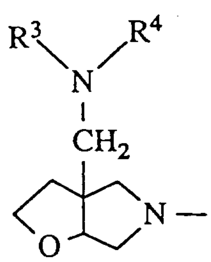

steht, worin

- $R^3$ für Wasserstoff, Methyl oder Ethyl,
- $R^4$ für Wasserstoff, Acyl mit 1 bis 3 Kohlenstoffatomen, Alkoxycarbonyl mit 1 bis 4 Kohlenstoffatomen im Alkylteil oder Methyl steht und
- A für N oder C-$R^5$ steht, worin
- $R^5$ für Wasserstoff, Halogen, Methyl, Alkenyl mit 2 bis 3 Kohlenstoffatomen, Alkinyl mit 2 bis 3 Kohlenstoffatomen, Hydroxy oder Methoxy steht oder auch gemeinsam mit $R^1$ eine Brücke der Struktur

$-O-CH_2-{}^*CH-CH_3$, $-S-CH_2-{}^*CH-CH_3$ oder $-CH_2-CH_2-{}^*CH-CH_3$

$-O-CH_2-N-R^6$,

worin $R^6$ Wasserstoff, Methyl oder Formyl bedeutet, bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren eine hohe antibakterielle Wirkung insbesondere gegenüber grampositiven Bakterien aufweisen.

Bevorzugt sind die Verbindungen der Formel (I),

in welcher

- $X^1$ für Fluor,
- $X^2$ für Wasserstoff, Amino, Methylamino, Hydroxy, Methoxy, Fluor, Chlor, Brom oder Methyl,
- $R^1$ für Alkyl mit 1 bis 3 Kohlenstoffatomen, Vinyl, Cycloalkyl mit 3 bis 4 Kohlenstoffatomen, 2-(cis- oder trans-)Fluorcyclopropyl, gegebenenfalls durch 1 oder 2 Fluoratome substituiertes Phenyl,
- $R^2$ für Wasserstoff, gegebenenfalls durch Amino, Methylamino oder Dimethylamino substituiertes Alkyl mit 1 bis 2 Kohlenstoffatomen oder (5-Methyl-2-oxo-1,3-dioxol-4-yl)-methyl,
- Z für einen Rest der Struktur

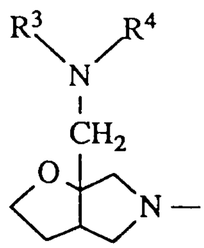
,
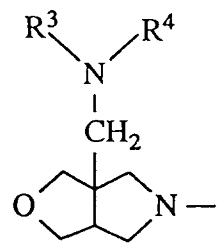
oder
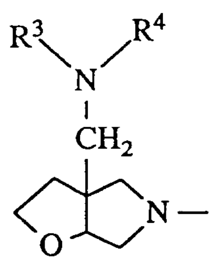

steht, worin

- $R^3$ für Wasserstoff oder Methyl,
- $R^4$ für Wasserstoff, Acyl mit 1 bis 2 Kohlenstoffatomen, Alkoxycarbonyl mit 1 bis 4 Kohlenstoffatomen im Alkylteil oder Methyl steht und
- A für N oder C-$R^5$ steht, worin
- $R^5$ für Wasserstoff, Fluor, Chlor, Methyl, Vinyl, Ethinyl oder Methoxy steht oder auch gemeinsam mit $R^1$ eine Brücke der Struktur

$-O-CH_2-{}^*CH-CH_3$ oder $-O-CH_2-N-CH_3$

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I),

in welcher

- $X^1$ für Fluor,
- $X^2$ für Wasserstoff, Amino oder Fluor,
- $R^1$ für Alkyl mit 1 bis 2 Kohlenstoffatomen, Cyclopropyl, 2-(cis- oder trans-)Fluorcyclopropyl, gegebenenfalls durch 1 oder 2 Fluoratome substituiertes Phenyl,
- $R^2$ für Wasserstoff oder Alkyl mit 1 bis 2 Kohlenstoffatomen,
- Z für einen Rest der Struktur

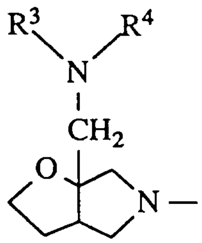
,
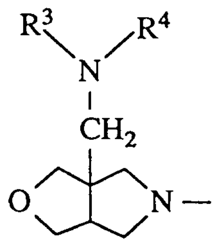
oder
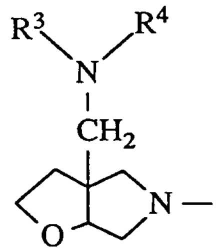

steht, worin

$R^3$ für Wasserstoff,

$R^4$ für Wasserstoff oder Alkoxcarbonyl mit 1 bis 4 Kohlenstoffatomen im Alkylteil steht und

A für N oder C-$R^5$ steht, worin

$R^5$ für Wasserstoff, Fluor, Chlor oder Methoxy steht oder auch gemeinsam mit $R^1$ eine Brücke der Struktur

$$-O-CH_2-{}^*CH-CH_3$$

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn man eine Verbindung der Formel (II)

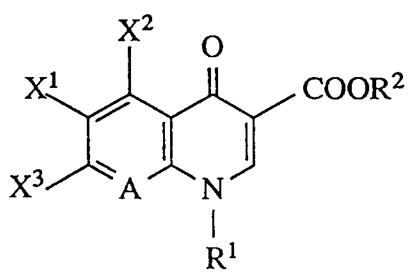
(II),

in welcher

A, $R^1$, $R^2$, $X^1$ und $X^2$ die oben angegebene Bedeutung haben, und

$X^3$ für Halogen, insbesondere für Fluor oder Chlor, steht,

mit Verbindungen der Formel (III)

Z'-H (III),

in welcher

Z' die oben für Z angegebene Bedeutung hat oder für einen Rest Z steht, dessen Aminogruppe gegebenenfalls durch eine Schutzgruppe blockiert sein kann,

gegebenenfalls in Gegenwart von Säurefängern umsetzt und gegebenenfalls vorhandene Schutzgruppen abspaltet.

Verwendet man beispielsweise 1-Cyclopropyl-6,7,8-trifluor-1,4-dihydro-4-oxo-3-chinolincarbonsäure und 1-(tert.-Butyloxycarbonylaminomethyl)-2-oxa-7-aza-bicyclo[3.3.0]octan als Ausgangsstoffe, so kann der Reaktionsverlauf durch folgendes Formelschema wiedergegeben werden:

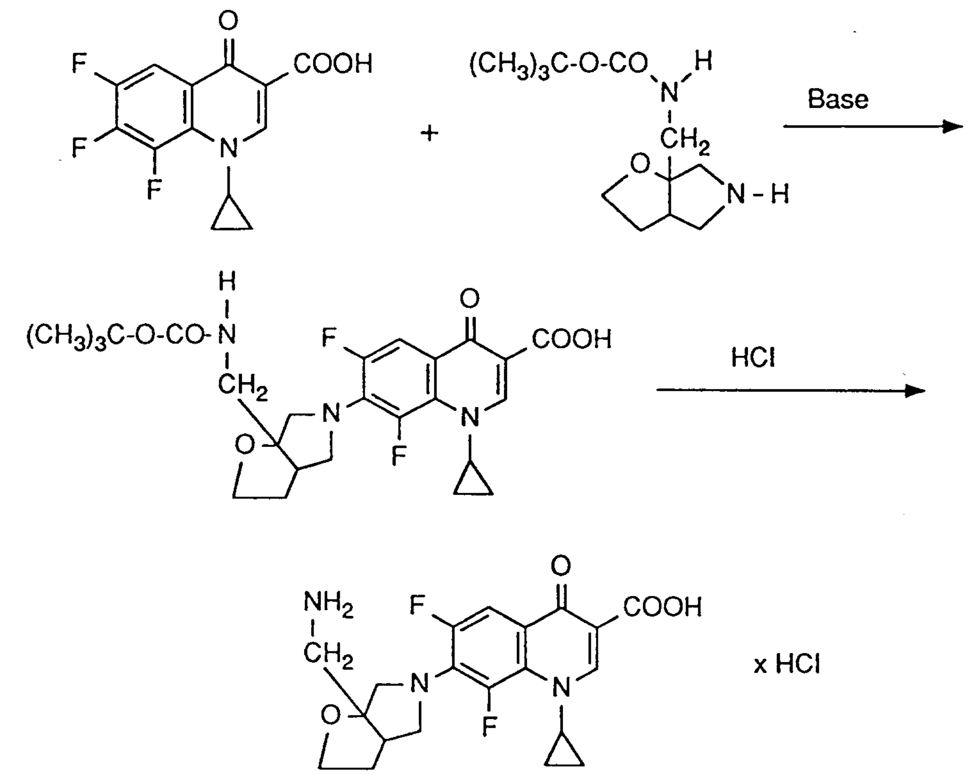


Die meisten der als Ausgangsstoffe verwendeten Verbindungen der Formel (II) sind bekannt oder können nach bekannten Methoden hergestellt werden. Als Beispiele seien genannt:

7-Chlor-1-cyclopropyl-6-fluor-1,4-dihydro-4-oxo-3-chinolincarbonsäure,
1-Cyclopropyl-6,7-difluor-1,4-dihydro-4-oxo-3-chinolincarbonsäure,
6-Chlor-1-cyclopropyl-7,8-difluor-1,4-dihydro-4-oxo-3-chinolincarbonsäure,
8-Chlor-1-cyclopropyl-6,7-difluor-1,4-dihydro-4-oxo-3-chinolincarbonsäure,
1-Cyclopropyl-6,7,8-trifluor-1,4-dihydro-4-oxo-3-chinolincarbonsäure,
5-Brom-1-cyclopropyl-6,7,8-trifluor-1,4-dihydro-4-oxo-3-chinolincarbonsäure,
5-Brom-1-(2,4-difluorphenyl)-6,7,8-trifluor-1,4-dihydro-4-oxo-3-chinolincarbonsäure,
1-Cyclopropyl-6,7-difluor-1,4-dihydro-8-methyl-4-oxo-3-chinolincarbonsäure,
6,7-Difluor-1-ethyl-1,4-dihydro-4-oxo-3-chinolincarbonsäure,
7-Chlor-6-fluor-1-ethyl-1,4-dihydro-4-oxo-3-chinolincarbonsäure,
7-Chlor-6-fluor-1,4-dihydro-1-(2-hydroxyethyl)-4-oxo-3-chinolincarbonsäure,
6,7-Difluor-1-(2-fluorethyl)-1,4-dihydro-4-oxo-3-chinolincarbonsäure,
7-Chlor-6-fluor-1,4-dihydro-1-methoxy-4-oxo-3-chinolincarbonsäure,
7-Chlor-6-fluor-1,4-dihydro-1-methylamino-4-oxo-3-chinolincarbonsäure,
6,7-Difluor-1,4-dihydro-4-oxo-1-phenyl-3-chinolincarbonsäure,
7-Chlor-1-cyclopropyl-6-fluor-1,4-dihydro-4-oxo-1,8-naphthyridin-3-carbonsäure,
7-Chlor-1-cyclopropyl-6-fluor-1,4-dihydro-4-oxo-1,8-naphthyridin-3-carbonsäureethylester,
1-Cyclopropyl-6,7,8-trifluor-1,4-dihydro-4-oxo-3-chinolincarbonsäureethylester,
9,10-Difluor-2,3-dihydro-3-methyl-7-oxo-7H-pyrido-[1,2,3-de][1,4]benzoxacin-6-carbonsäure,
8,9-Difluor-6,7-dihydro-5-methyl-1-oxo-1H,5H-benzo[i,j]-chinolicin-2-carbonsäure,
7-Chlor-6-fluor-1-phenyl-1,4-dihydro-4-oxo-1,8-naphthyridin-3-carbonsäure,
7-Chlor-6-fluor-1-(4-fluorphenyl)-1,4-dihydro-4-oxo-1,8-naphthyridin-3-carbonsäureethylester,
6,7,8-Trifluor-1,4-dihydro-1-methylamino-4-oxo-3-chinolincarbonsäure,
1-Amino-6,7,8-trifluor-1,4-dihydro-4-oxo-3-chinolincarbonsäure,
6,7,8-Trifluor-1,4-dihydro-1-dimethylamino-4-oxo-3-chinolincarbonsäure,
6,7-Difluor-1-(4-fluorphenyl)-1,4-dihydro-8-methyl-4-oxo-3-chinolincarbonsäure,
7-Chlor-6-fluor-1-(4-fluorphenyl)-1,4-dihydro-4-oxo-3-chinolincarbonsäure,
7-Chlor-6-fluor-1-(2,4-difluorphenyl)-1,4-dihydro-4-oxo-3-chinolincarbonsäure,
6,7,8-Trifluor-1-(4-fluorphenyl)-1,4-dihydro-4-oxo-3-chinolincarbonsäure,

1-Cyclopropyl-6,7-difluor-1,4-dihydro-5-methyl-4-oxo-3-chinolincarbonsäure,
7-Chlor-1-cyclopropyl-6-fluor-1,4-dihydro-5-methyl-4-oxo-1,8-naphthyridin-3-carbonsäure,
7-Chlor-6-fluor-1-(2,4-difluorphenyl)-1,4-dihydro-5-methyl-4-oxo-1,8-naphthyridin-3-carbonsäure,
6,7-Difluor-1,4-dihydro-1-(3-oxetanyl)-4-oxo-3-chinolincarbonsäure,
6,7,8-Trifluor-1,4-dihydro-1-(3-oxetanyl)-4-oxo-3-chinolincarbonsäure,
1-(Bicyclo[1.1.1]pent-1-yl)-6,7,8-trifluor-1,4-dihydro-4-oxo-3-chinolincarbonsäure,
7-Chlor-1-(1,1-dimethylpropargyl)-6-fluor-1,4-dihydro-4-oxo-1,8-naphthyridin-3-carbonsäure,
6,7,8-Trifluor-1-(2,4-difluorphenyl)-1,4-dihydro-4-oxo-3-chinolincarbonsäure,
6,7,8-Trifluor-1,4-dihydro-4-oxo-1-phenyl-3-chinolincarbonsäure,
7-Chlor-1-ethyl-6-fluor-1,4-dihydro-4-oxo-1,8-naphthyridin-3-carbonsäure,
6,7-Difluor-1,4-dihydro-4-oxo-1-vinyl-3-chinolincarbonsäure,
1-Cyclopropyl-5,6,7,8-tetrafluor-1,4-dihydro-4-oxo-3-chinolincarbonsäure,
5-Amino-1-cyclopropyl-6,7,8-trifluor-1,4-dihydro-4-oxo-3-chinolincarbonsäure,
1-Cyclopropyl-6,7,8-trifluor-1,4-dihydro-5-hydroxy-4-oxo-3-chinolincarbonsäure,
1-Cyclopropyl-6,7-difluor-1,4-dihydro-8-methoxy-4-oxo-3-chinolincarbonsäure,
7-Chlor-1-(2,4-difluorphenyl)-6-fluor-1,4-dihydro-4-oxo-1,8-naphthyridin-3-carbonsäure,
7-Chlor-1-(2,4-difluorphenyl)-6-fluor-1,4-dihydro-4-oxo-1,8-naphthyridin-3-carbonsäureethylester,
1-Cyclopropyl-6,7-difluor-1,4-dihydro-4-oxo-8-vinyl-3-chinolincarbonsäure,
1-Cyclopropyl-8-ethinyl-6,7-difluor-1,4-dihydro-4-oxo-3-chinolincarbonsäure,
6,7,8-Trifluor-1-(cis- und trans-2-fluorcyclopropyl)-1,4-dihydro-4-oxo-3-chinolincarbonsäure,
8-Chlor-6,7-difluor-1-(cis- und trans-2-fluorcyclopropyl)-1,4-dihydro-4-oxo-3-chinolincarbonsäure,
7-Chlor-6-fluor-1-(cis- und trans-2-fluorcyclopropyl)-1,4-dihydro-4-oxo-1,8-naphthyridin-3-carbonsäure,
6,7-Difluor-1-(cis- und trans-2-fluorcyclopropyl)-1,4-dihydro-4-oxo-3-chinolincarbonsäure.

Die als Ausgangsverbindungen benötigten bicyclischen Amine der Formel (III) sind neu. Sie können nach folgenden Verfahren hergestellt werden.

Die Beispiele sind illustrativ, aber keineswegs einschränkend gemeint.

Man setzt 2,3- bzw. 2,5-Dihydrofuran (1), welches an der Doppelbindung durch eine elektronenanziehende Gruppe, die sich in eine Aminomethylgruppe überführen läßt, substituiert ist, mit einem Azomethinylid der Struktur (2) um. Das erhaltene Cycloadditionsprodukt (3) wird anschließend zum Aminomethylderivat (4) umgesetzt, aus welchem die Schutzgruppe $S_2$ selektiv abgespalten wird. Durch Derivatisierung der Aminomethylgruppe in (4) lassen sich über (6) bzw. (8) die entsprechenden Substitutionsprodukte (7) bzw. (9) aufbauen.

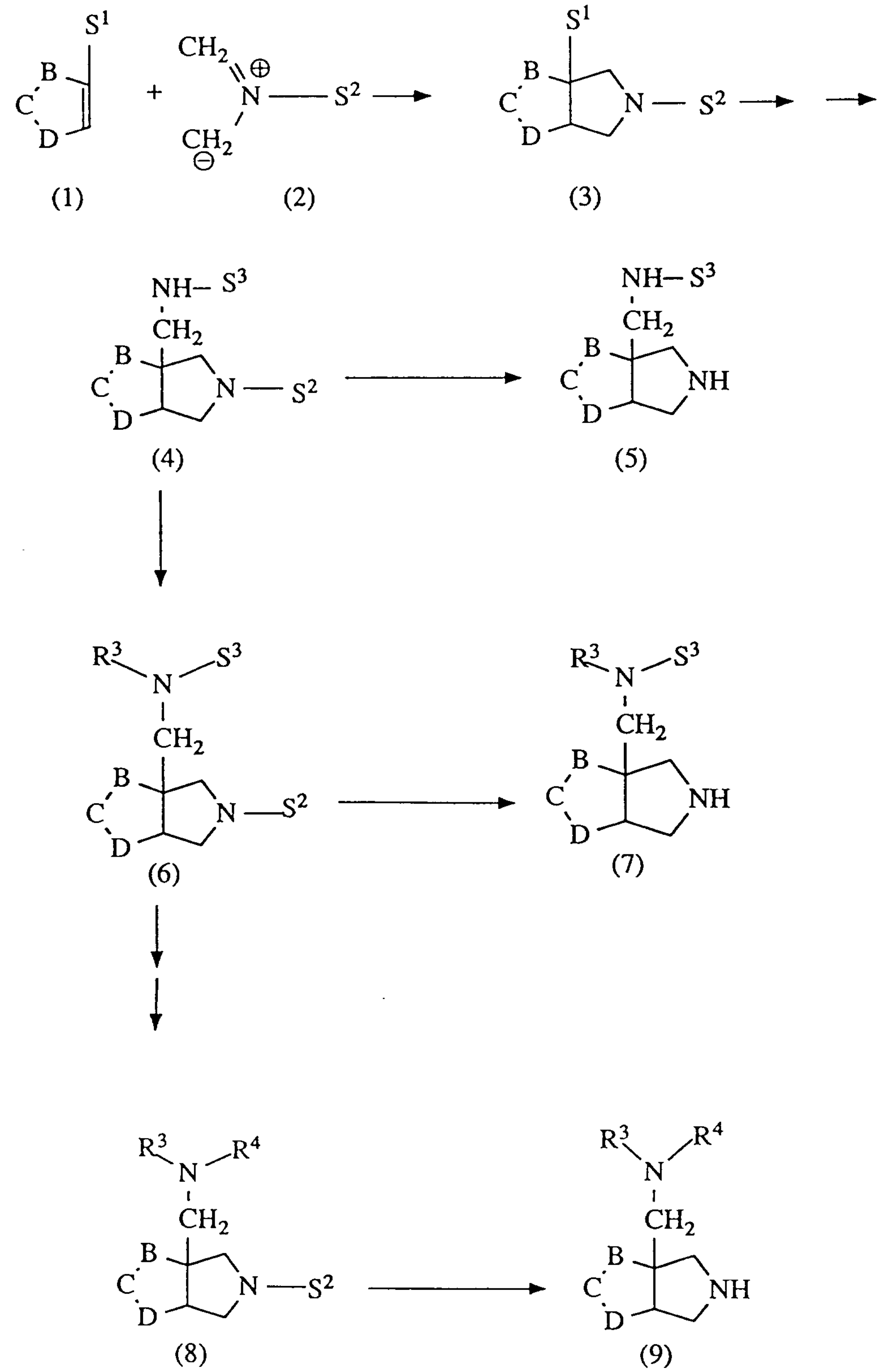

(1) (2) (3) (4) (5) (6) (7) (8) (9)

B, C, D: $CH_2$ oder Sauerstoff, wobei eine der Gruppen B, C oder D Sauerstoff sein muß,
$S^1$: CN, $CO-NH_2$, $CO-NR^3R^4$, COO-Alkyl($C_1$-$C_3$), $CO-CF_3$, $CO-CH_3$,
$S^2$: $CH_2-C_6H_5$, $CO-C_6H_5$,
$S^3$: CO-O-Alkyl($C_1$-$C_4$).

Setzt man beispielsweise 2,3-Dihydrofuran-4-carbonsäuremethylester mit N-Benzyl-N-methoxymethyl-trimethylsilylmethylamin um, so kann die Umsetzung zum 5-tert.-Butoxycarbonylaminomethyl-2-oxa-7-azabicyclo[3.3.0]octan durch folgendes Reaktionsschema wiedergegeben werden:

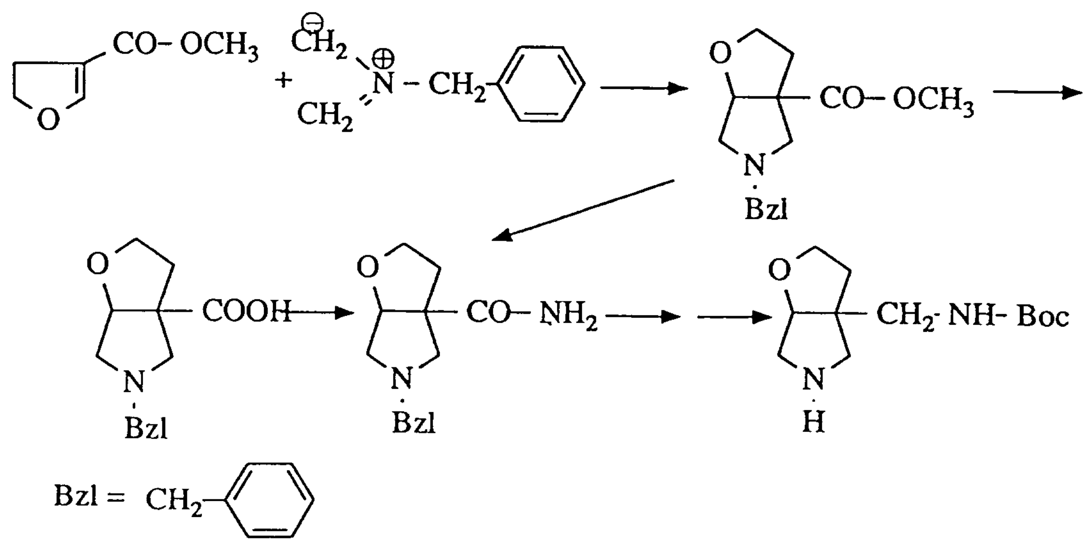

Als Beispiele für Verbindungen der Formel (III), die sowohl als Racemate als auch als enantiomeren- oder diastereomerenreine Verbindungen eingesetzt werden können, seien genannt:
1-Aminomethyl-2-oxa-7-aza-bicyclo[3.3.0]octan,
1-Methylaminomethyl-2-oxa-7-aza-bicyclo[3.3.0]octan,
1-Dimethylaminomethyl-2-oxa-7-aza-bicyclo[3.3.0]octan,
1-Ethylaminomethyl-2-oxa-7-aza-bicyclo[3.3.0]octan,
1-Formylaminomethyl-2-oxa-7-aza-bicyclo[3.3.0]octan,
1-Acetylaminomethyl-2-oxa-7-aza-bicyclo[3.3.0]octan,
1-Methoxycarbonylaminomethyl-2-oxa-7-aza-bicyclo[3.3.0]octan,
1-Ethoxycarbonylaminomethyl-2-oxa-7-aza-bicyclo[3.3.0]octan,
1-tert.-Butoxycarbonylaminomethyl-2-oxa-7-aza-bicyclo[3.3.0]octan,
1-Aminomethyl-3-oxa-7-aza-bicyclo[3.3.0]octan,
1-Methylaminomethyl-3-oxa-7-aza-bicyclo[3.3.0]octan,
1-Dimethylaminomethyl-3-oxa-7-aza-bicyclo[3.3.0]octan,
1-Ethylaminomethyl-3-oxa-7-aza-bicyclo[3.3.0]octan,
1-Formylaminomethyl-3-oxa-7-aza-bicyclo[3.3.0]octan,
1-Acetylaminomethyl-3-oxa-7-aza-bicyclo[3.3.0]octan,
1-Methoxycarbonylaminomethyl-3-oxa-7-aza-bicyclo[3.3.0]octan,
1-Ethoxycarbonylaminomethyl-3-oxa-7-aza-bicyclo[3.3.0]octan,
1-tert.-Butoxycarbonylaminomethyl-3-oxa-7-aza-bicyclo[3.3.0]octan,
5-Aminomethyl-2-oxa-7-aza-bicyclo[3.3.0]octan,
5-Methylaminomethyl-2-oxa-7-aza-bicyclo[3.3.0]octan,
5-Dimethylaminomethyl-2-oxa-7-aza-bicyclo[3.3.0]octan,
5-Ethylaminomethyl-2-oxa-7-aza-bicyclo[3.3.0]octan,
5-Formylaminomethyl-2-oxa-7-aza-bicyclo[3.3.0]octan,
5-Acetylaminomethyl-2-oxa-7-aza-bicyclo[3.3.0]octan,
5-Acetylaminomethyl-2-oxa-7-aza-bicyclo[3.3.0]octan,
5-Methoxycarbonylaminomethyl-2-oxa-7-aza-bicyclo[3.3.0]octan,
5-Ethoxycarbonylaminomethyl-2-oxa-7-aza-bicyclo[3.3.0]octan,
5-tert.-Butoxycarbonylaminomethyl-2-oxa-7-aza-bicyclo[3.3.0]octan.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol, der Verbindung (III) ein.

Freie Aminogruppen können während der Umsetzung durch eine geeignete Aminoschutzgruppe, z.B. durch den tert.-Butoxycarbonylrest oder als Azomethingruppe, geschützt und nach Beendigung der Reaktion durch Behandlung mit einer geeigneten Säure wie Chlorwasserstoffsäure oder Trifluoressigsäure wieder freigesetzt werden.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20 bis 200 °C, vorzugsweise etwa 60 bis 120 °C, umgesetzt. Das entstehende Reaktionswasser kann auch durch azeotrope Destillation mit Chloroform, Tetrachlormethan, Benzol oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten (5-Methyl-2-oxo-1,3-dioxol-4-yl-methyl)-ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit 4-Brommethyl- oder 4-Chlormethyl-5-methyl-1,3-dioxol-2-on in einem Lösungsmittel wie Dimethylformamid, Dimethylacetamid, N-Methylpyrrolidon, Dimethylsulfoxid oder Tetramethylharnstoff bei Temperaturen von etwa 0 bis 100 °C, vorzugsweise 0 bis 50 °C, erhalten.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die in der folgenden Tabelle aufgeführten Wirkstoffe hergestellt werden, die als Racemate oder als enantiomerenreine oder diastereomerenreine Verbindungen vorliegen können:

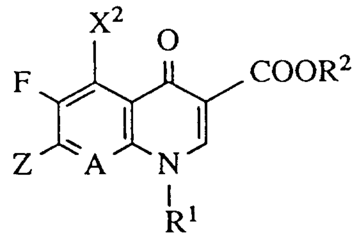

| $R^1$ | $R^2$ | $X^2$ | Z | A |
|---|---|---|---|---|
| 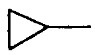 | H | $CH_3$ | 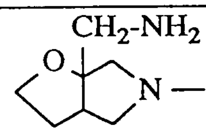 | N |
| 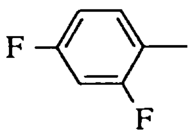 | H | H | 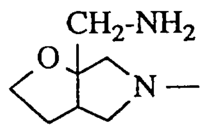 | N |
| 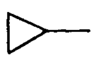 | H | Cl | 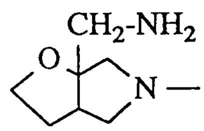 | C-Cl |
| 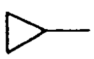 | H | $NH_2$ | 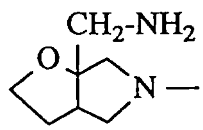 | C-$OCH_3$ |
| 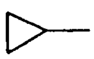 | H | H | 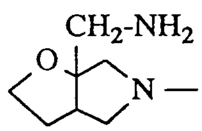 | C-$CH_3$ |
| 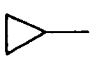 | H | $CH_3$ | 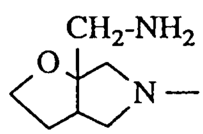 | CH |
| 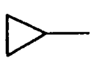 | H | $CH_3$ | 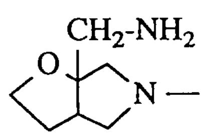 | CF |
| 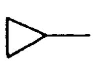 | H | H | 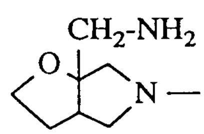 | C-Br |

| $R^1$ | $R^2$ | $X^2$ | Z | A |
|---|---|---|---|---|
| 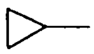 | H | Br | 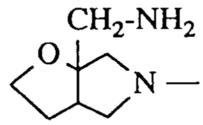 | CF |
| 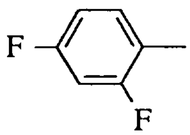 | H | Br | 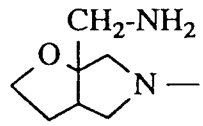 | CF |
| 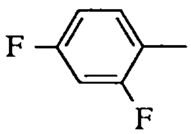 | H | F | 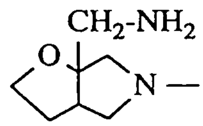 | CF |
| 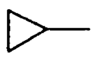 | H | F | 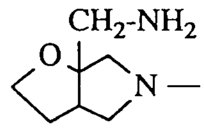 | CH |
| 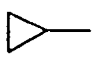 | $C_2H_5$ | H | 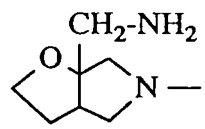 | CF |
| 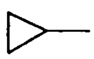 | $CH_2CH_2NH_2$ | H | 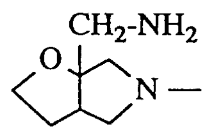 | CF |
| 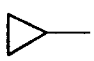 | $CH_2CH_2$-OH | H | 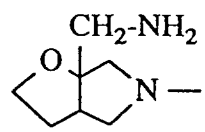 | CCl |
| $C_2H_5$ | H | H | 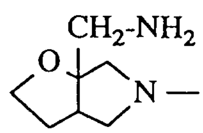 | CF |
| 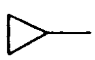 | H | $NH_2$ | 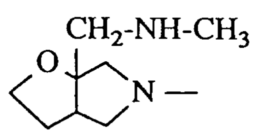 | CF |
| 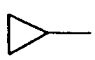 | H | H | 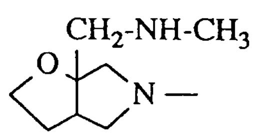 | CF |

| $R^1$ | $R^2$ | $X^2$ | Z | A |
|---|---|---|---|---|
| 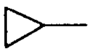 | H | H | 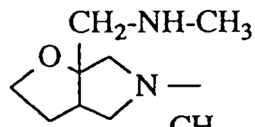 | CF |
| 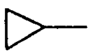 | H | H | 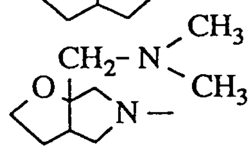 | CF |
| 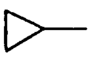 | H | H | 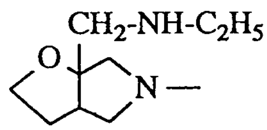 | CF |
| 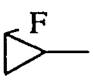 | H | H | 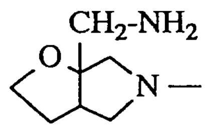 | CF |
| 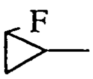 | H | $NH_2$ | 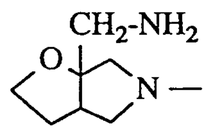 | CF |
| 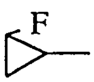 | H | H | 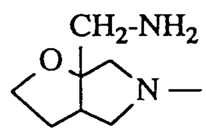 | CCl |
| 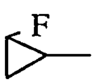 | H | H | 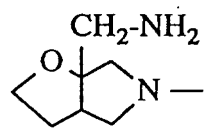 | N |
| 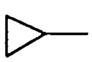 | H | H | 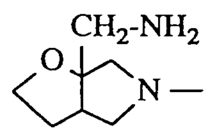 | $C-CH=CH_2$ |
| 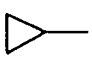 | H | H | 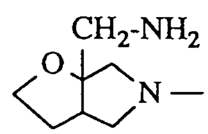 | $C-CH\equiv CH$ |
| 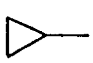 | H | $NH_2$ | 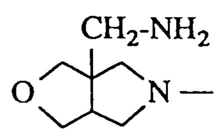 | CF |

| $R^1$ | $R^2$ | $X^2$ | Z | A |
|---|---|---|---|---|
| 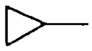 | H | H | 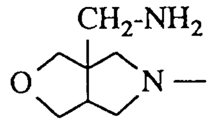 | N |
| 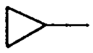 | H | H | 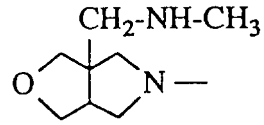 | CF |
| 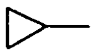 | H | H | 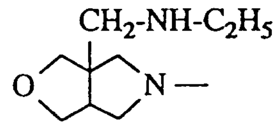 | CF |
| 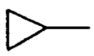 | H | H | 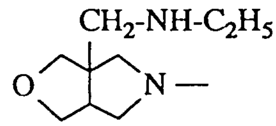 | N |
| 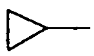 | H | H | 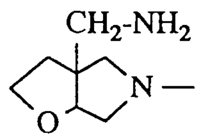 | CF |
| 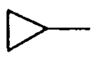 | H | H | 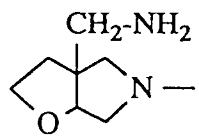 | CCl |
| 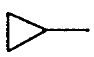 | H | H | 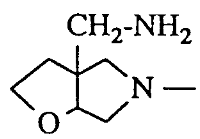 | CH |
| 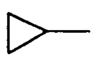 | H | H | 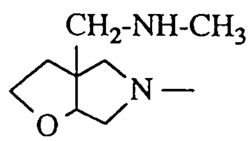 | CCl |
| 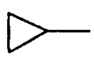 | H | H | 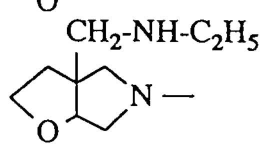 | CCl |
| 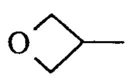 | H | H | 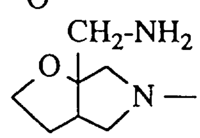 | CF |

| $R^1$ | $R^2$ | $X^2$ | Z | A |
|---|---|---|---|---|
| $F-CH_2CH_2-$ | H | H | 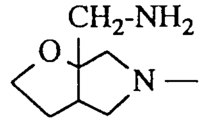 | CF |
| $CH_3-NH-$ | H | H | 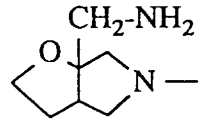 | CF |
| 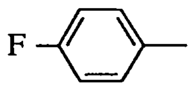 | H | H | 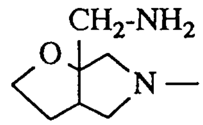 | CF |
| 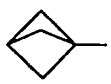 | H | H | 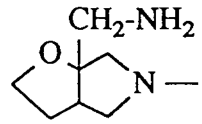 | CF |

Die erfindungsgemäßen Verbindungen wirken stark antibiotisch und zeigen bei geringer Toxizität ein breites antibakterielles Spektrum gegen grampositive und gramnegative Keime, insbesondere gegen Enterobakterien; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf resistente Keime aus. Bei Bakterien, die kein nachweisbares Wachstum zeigen, wirken die Verbindungen stärker bakterizid als bisher bekannte Substanzen Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Pseudomonas aeruginosa, Enterococcus faecalis und Escherichia coli beobachtet werden.

Auch gegenüber Bakterien, die gegenüber vergleichbaren Substanzen als weniger empfindlich eingestuft werden, insbesondere resistente Staphylococcus aureus, Escherichia coli, Pseudomonas aeruginosa und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human-und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die erfindungsgemäßen Verbindungen können auch mit $\beta$-Lactamderivaten wie zum Beispiel Cephalosporinen oder Penicillinen über kovalente Bindungen zu Dual-Action-Derivaten kombiniert werden.

Die minimalen Hemmkonzentrationen (MHK) wurden per Reihenverdünnungsverfahren auf Iso-Sensitest Agar (Oxoid) bestimmt. Für jede Prüfüngssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel

enthielt. Die beimpften Agarplatten wurden bei 37 °C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich zu 1-Cyclopropyl-6,8-difluor-1,4-dihydro-7-(cis-2-oxa-5,8-diazabicyclo[4.3.0]non-8-yl)-4-oxo-3-chinolincarbonsäure-Hydrochlorid (Referenzbeispiel A) und 8-Chlor-1-cyclopropyl-6-fluor-1,4-dihydro-7-(cis-2-oxa-5,8-diazabicyclo[4.3.0]non-8-yl)-4-oxo-3-chinolincarbonsäure-Hydrochlorid (Referenzbeispiel B) aufgeführt.

MHK-Werte

| Keime | | Beispiel 1B | Beispiel 2B | Referenzbeispiel A | Referenzbeispiel B |
|---|---|---|---|---|---|
| Escherichia coli | 455/7 | 2 | 1 | 4 | 2 |
| Klebsiella pneumoniae | 8085 | 0,06 | 0,03 | 0,06 | 0,06 |
| Enterobacter cloaceae | 2427 | 0,06 | 0,06 | 0,125 | 0,25 |
| Enterobacter aerog. | ICB 5240 | 8 | 8 | 32 | 16 |
| Morganella morganii | 932 | 0,03 | 0,03 | 0,125 | 0,06 |
| Providencia sp. | 12012 | 0,06 | 0,03 | 0,125 | 0,06 |
| Micrococcus luteus | 9341 | 0,25 | 0,125 | 0,5 | 0,25 |
| Staphylococcus aureus | ICB 25701 | 1 | 0,25 | 2 | 0,5 |
| | 1756 | ≤0,015 | 0,0039 | 0,06 | 0,015 |
| | 133 | 0,06 | 0,015 | 0,06 | 0,03 |
| Enterococcus faecalis | 27101 | 0,06 | 0,03 | 0,25 | 0,125 |
| | 9790 | 0,125 | 0,06 | 0,25 | 0,125 |
| Pseudomonas aeruginosa | ICB 308029 | 16 | 32 | 64 | 64 |

Herstellung der Zwischenprodukte

Beispiel A

a) 7-Benzyl-1-cyano-2-oxa-7-azabicyclo[3.3.0]octan
Man legt 14,4g (0,15 mol) 4,5-Dihydrofuran-2-carbonsäurenitril in 300 ml absolutem Methylenchlorid vor, setzt 1,8 g Triethylamin und 1,8 g Trifluoressigsäure hinzu und erwärmt auf 40 °C. Dazu tropft man 42,6 g (0,15 mol) N-Benzyl-N-methoxymethyl-N-trimethylsilylmethylamin und rührt 15 Stunden bei 40 °C. Der Ansatz wird mit 50 ml gesättigter Natriumhydrogencarbonatlösung gewaschen, über $MgSO_4$ getrocknet, eingeengt und zweimal destilliert.
Ausbeute: 9,3 g (22,5 % der Theorie)
Siedepunkt: 120-125 °C/0,06 mbar
Das Produkt ist gaschromatographisch 83 %ig.
b) 1-Aminomethyl-7-benzyl-2-oxa-7-aza-bicycl[3.3.0]octan
Zu 2 g Lithiumaluminiumhydrid in 100 ml absolutem Tetrahydrofuran tropft man 10,2 g (40 mmol, 90 %ig) 7-Benzyl-1-cyano-2-oxa-7-aza-bicyclo[3.3.0]octan und rührt anschließend 15 Stunden unter Rückfluß. Man tropft je 2 ml Wasser, 15 %ige Kalilauge und wieder Wasser hinzu, saugt die anorganischen Salze ab, engt das Filtrat ein und destilliert
Ausbeute: 7,7 g (83 % der Theorie).
c) 7-Benzyl-1-tert.-butoxycarbonylaminomethyl-2-oxa-7-aza-bicyclo[3.3.0]octan
Zu 10,4 g (44,7 mmol) 1-Aminomethyl-7-benzyl-2-oxa-7-azabicyclo[3.3.0]octan in 56 ml tert.-Butanol gibt man 2 g NaOH in 45 ml Wasser und tropft dann 10,2 g (47 mmol) Pyrokohlensäuredi-tert.-butylester hinzu. Man rührt 15 Stunden bei Raumtemperatur, extrahiert mit Chloroform, trocknet über Kaliumcarbonat, engt ein und destilliert.
Ausbeute: 12,2 g (82 % der Theorie)
Siedepunkt: 170 °C/0,15 mbar
Das Produkt ist gaschromatographisch 94 %ig.
d) 1-tert.-Butoxycarbonylaminomethyl-2-oxa-7-aza-bicyclo[3.3.0]octan
Man hydriert 12,2 g (34,5 mmol) 7-Benzyl-1-tert.-butoxycarbonylaminomethyl-2-oxa-7-azabicyclo[3.3.0]-octan in 200 ml Ethanol an 7 g Palladium-Aktivkohle (10 % Pd) bei 100 °C und 100 bar. Man saugt den Katalysator ab, engt das Filtrat ein und destilliert.
Ausbeute: 5,7 g (68 % der Theorie)
Siedepunkt: 120-130 °C/0,01 mbar.

Beispiel B

1-Aminomethyl-3-oxa-7-azabicyclo[3.3.0]octan

a) 3-Cyano-2,5-dihydrofuran
Man legt 17,2 g (0,2 mol) 3-Oxotetrahydrofuran und 1 g Zinkiodid in 100 ml Benzol vor und tropft 24 g (0,24 mol) Trimethylsilylcyanid hinzu. Man rührt über Nacht bei Raumtemperatur, setzt 300 ml Pyridin hinzu und tropft 92 g (0,6 mol) Phosphoroxychlorid dazu. Anschließend wird 20 Stunden unter Rückfluß erhitzt. Man gießt auf Eis, extrahiert mit Diethylether, wäscht die Etherlösungungen mit Kochsalzlösung, trocknet über Magnesiumsulfat, engt ein und destlliert.
Ausbeute: 10 g (24 % der Theorie)
Siedepunkt: 80 °C/20 mbar
Das Produkt enthält nach GC-MS 45 % 3-Cyano-2,5-dihydrofuran, 17 % 3-Cyano-4,5-dihydrofuran und 37 % 3-Chlor-3-cyano-tetrahydrofuran.
b) 7-Benzyl-1-cyano-3-oxa-7-azabicyclo[3.3.0]octan
Eine Lösung von 5,2 g (24,6 mmol), 46 %ig) 3-Cyano-2,5-dihydrofuran in 50 ml absolutem Methylenchlorid wird mit 0,3 g Triethylamin und 0,3 g Trifluoressigsäure versetzt, die Mischung unter Rückfluß erhitzt und dann 7 g (29,5 mmol) N-Benzyl-N-methoxymethyl-N-(trimethylsilylmethyl)-amin zugetropft Anschließend erhitzt man zwei Stunden unter Rückfluß, wäscht die lösung mit gesättigter Natriumhydrogencarbonatlösung, trocknet über Magnesiumsulfat, engt ein und destilliert.
Ausbeute: 4,5 g (80 % der Theorie).
Siedepunkt: 140-145 °C/0,03 mbar
c) 1-Aminomethyl-7-benzyl-3-oxa-7-azabicyclo[3.3.0]octan
Man tropft 3,7 g (15,9 mmol (7-Benzyl-1-cyano-3-oxa-7-azabicyclo[3.3.0]octan in 5 ml absolutem Tetrahydrofuran zu einer Mischung von 1,5 g Lithiumaluminiumhydrid in 100 ml absolutem Tetrahydrofüran und erhitzt 15 Stunden unter Rückfluß. Man zersetzt mit je 1,5 ml Wasser, 15 %iger Natronlauge und wieder Wasser, saugt ab und kocht die Aluminiumsalze zweimal mit 100 ml Tetrahydrofuran aus. Die Tetrahydrofuranlösungen werden eingeengt und der Rückstand destilliert.
Ausbeute: 3,2 g (86 % der Theorie)
Siedepunkt: 125-130 °C/0,04 mbar
d) 1-Aminomethyl-3-oxa-7-azabicyclo[3.3.0]octan
2,8 g (12,1 mmol) 1-Aminomethyl-7-azabicyclo[3.3.0]octan werden in 50 ml Ethanol gelöst und an 0,3 g Palladium-Aktivkohle bei 100 °C und 100 bar hydriert Man saugt den Katalysator ab, engt das Filtrat ein und destilliert.
Ausbeute: 1,5 g (87 % der Theorie)
Siedepunkt: 70-72 °C/0,03 mbar.

Beispiel 1

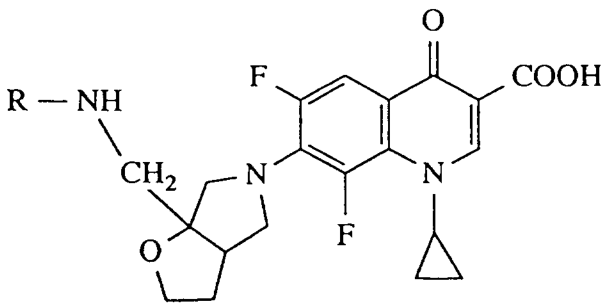

A: R = $(CH_3)_3C$-O-CO
B: R = H x HCl

A: Eine Mischung von 1,42 g (5 mmol) 1-Cyclopropyl-6,7,8-trifluor-1,4-dihydro-4-oxo-3-chinolincarbonsäure in 15 ml Acetonitril und 7,5 ml Dimethylformamid mit 560 mg (5 mmol) 1,4-Diazabicyclo[2.2.2]-octan und 1,3 g (5,4 mmol) 1-tert.-Butoxycarbonylaminomethyl-2-oxa-7-azabicyclo[3.3.0]octan wird 1 Stunde unter Rückfluß erhitzt. Die Suspension wird abgekühlt, der Niederschlag abgesaugt, mit Wasser gewaschen und aus Acetonitril umkristallisiert.
Ausbeute: 1,2 g (48,5 % der Theorie) 7-(1-tert.-Butoxycarbonylaminomethyl-2-oxa-7-azabicyclo[3.3.0]oct-7-yl)-1-cyclopropyl-6,8-difluor-1,4-dihydro-4-oxo-3-chinolincarbonsäure,
Schmelzpunkt: 188-190 °C (unter Zersetzung).
B: 1,1 g (2,2, mmol) 7-(1-tert.-Butoxycarbonylaminomethyl-2-oxa-7-azabicyclo[3.3.0]oct-7-yl)-1-cyclopropyl-6,8-difluor-1,4-dihydro-4-oxo-3-chinolincarbonsäure werden in 30 ml halbkonzentrierter Salzsäure in der Wärme gelöst, die lösung filtriert und bei 70 °C und 12 mbar eingeengt. Der Rückstand wird mit 40 ml Ethanol versetzt, die Mischung in Eis gekühlt, das ausgefallene Salz abgesaugt, mit Ethanol gewaschen und bei 100 °C im Hochvakuum getrocknet.
Ausbeute: 690 mg (72 % der Theorie) 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäure-Hydrochlorid,
Schmelzpunkt: 187-204 °C (unter Zersetzung).

Beispiel 2

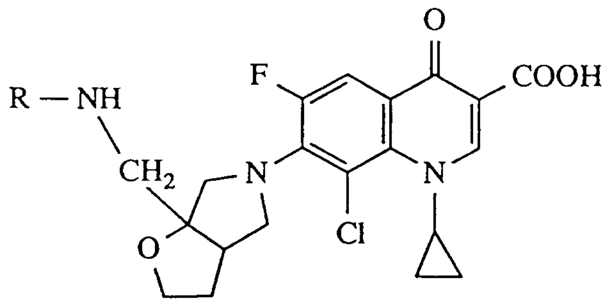

A: R = $(CH_3)_3C$-O-CO
B: R = H x $CF_3COOH$

A: Analog Beispiel 1A setzt man mit 8-Chlor-1-cyclopropyl-6,7-difluor-1,4-dihydro-4-oxo-3-chinolincarbonsäure zu 7-(1-tert.-Butoxycarbonylaminomethyl-2-oxa-7-aza-bicyclo[3.3.0]oct-7-yl)-8-chlor-1-cyclopropyl-6-fluor-1,4-dihydro-4-oxo-3-chinolincarbonsäure (88 % der Theorie) vom Schmelzpunkt 111-113 °C (umkristallisiert aus Ethanol) um.

B: 2,2 g (4,2 mmol) 7-(1-tert.-Butoxycarbonylaminomethyl-2-oxa-7-aza-bicyclo[3.3.0]oct-7-yl)-8-chlor-1-cyclopropyl-6-fluor-1,4-dihydro-4-oxo-3-chinolincarbonsäure werden in 5 ml Trifluoressigsäure gelöst, die lösung nach 12-stündigem Stehen bei Raumtemperatur eingedampft und das zurückbleibende gelbe Öl mehrmals mit Methylenchlorid im Ultraschallbad behandelt bis es kristallisierte. Das Salz wurde abgesaugt, mit Methylenchlorid gewaschen und bei 100 °C im Hochvakuum getrocknet.

Ausbeute: 1,27 g (56 % der Theorie) 7-(1-Aminomethyl-2-oxa-7-aza-bicyclo[3.3.0]oct-7-yl)-8-chlor-1-cyclopropyl-6-fluor-1,4-dihydro-4-oxo-3-chinolincarbonsäure-Trifluoracetat,
Schmelzpunkt: 230-233 °C (unter Zersetzung).

Beispiel 3

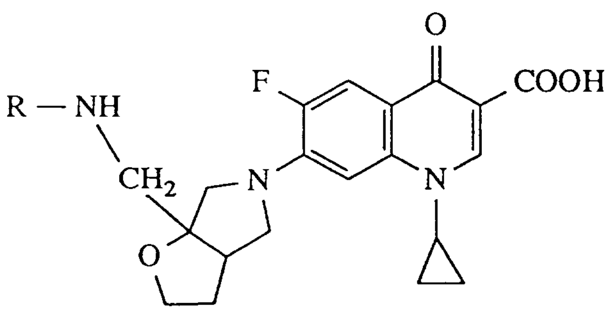

A: R = $(CH_3)_3C$-O-CO
B: R = H x HCl

A: Analog Beispiel 1A setzt man mit 1-Cyclopropyl-6,7-difluor-1,4-dihydro-4-oxo-3-chinolincarbonsäure zu 7-(1-tert.-Butoxycarbonylaminomethyl-2-oxa-7-aza-bicyclo[3.3.0]oct-7-yl)-1-cyclopropyl-6-fluor-1,4-dihydro-4-oxo-3-chinolincarbonsäure (98 % der Theorie) vom Schmelzpunkt 235-237 °C (umkristallisiert aus Glykolmonomethylether) um.

B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-1-cyclopropyl-6-fluor-1,4-dihydro-4-oxo-3-chinolincarbonsäure-Hydrochlorid vom Schmelzpunkt 223-226 °C (unter Zersetzung) umgesetzt.

Beispiel 4

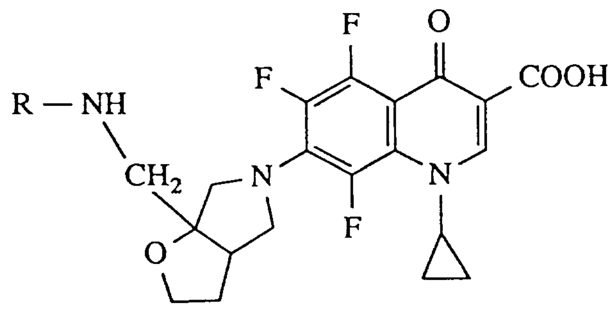

A: R = $(CH_3)_3C$-O-CO
B: R = H x HCl

A: Analog Beispiel 1A setzt man mit 1-Cyclopropyl-5,6,7,8-tetrafluor-1,4-dihydro-4-oxo-3-chinolincarbonsäure zu 7-(1-tert.-Butoxycarbonylaminomethyl-2-oxa-7-aza-bicyclo[3.3.0]oct-7-yl)-1-cyclopropyl-5,6,8-trifluor-1,4-dihydro-4-oxo-3-chinolincarbonsäure (95 % der Theorie) vom Schmelzpunkt 226-228 °C (unter Zersetzung) (umkristallisiert aus Glykolmonomethylether) um.

B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-1-cyclopropyl-5,6,8-trifluor-1,4-dihydro-4-oxo-3-chinolincarbonsäure-Hydrochlorid vom Schmelzpunkt 175-177 °C (unter Zersetzung) umgesetzt.

Beispiel 5

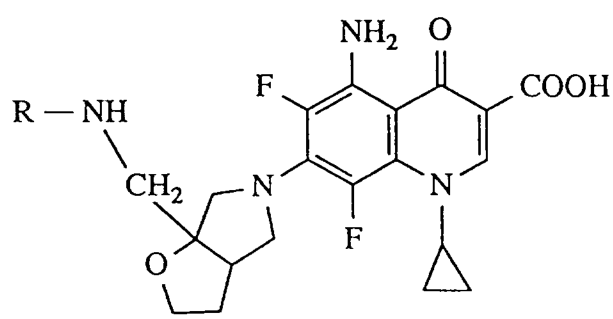

A: R = $(CH_3)_3C$-O-CO
B: R = H x HCl

A: 1 g (1,9 mmol) 7-(1-tert.-Butoxycarbonylaminomethyl-2-oxa-7-aza-bicyclo[3.3.0]oct-7-yl)-1-cyclopropyl-5,6,8-trifluor-1,4-dihydro-4-oxo-3-chinolincarbonsäure werden in 30 ml Dimethylsulfoxid gelöst und bei 130-140 °C während 5 Stunden ein Ammoniakstrom eingeleitet. Wenn dünnschichtchromatographisch (Kieselgel; Methylenchlorid/Methanol/17 % wäßriges Ammoniak 150:20:1) keine Ausgangsverbindung mehr nachweisbar ist, wird im Hochvakuum eingedampft, der Rückstand mit 30 ml Wasser verrührt und die Suspension über Nacht stehengelassen. Der ausgefallene Niederschlag wird abgesaugt, mit Wasser gewaschen und getrocknet. Das erhaltene Rohprodukt (580 mg) wird aus Glykolmonomethylether umkristallisiert.

Ausbeute: 290 mg (29 % der Theorie) 5-Amino-7-(1-tert.-butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäure vom Schmelzpunkt 181-183 °C (unter Zersetzung).

B: Analog Beispiel 1B wird das Produkt aus der Stufe A mit halbkonzentrierter Salzsäure zu 5-Amino-7-(1-aminomethyl-2-oxa-7-aza-bicyclo[3.3.0]oct-7-yl)-1-cyclopropyl-6,8-difluor-1,4-dihydro-4-oxo-3-chinolincarbonsäure-Hydrochlorid vom Schmelzpunkt 262-263 °C (unter Zersetzung) umgesetzt.

FAB-MS: m/e 421 $[(M+H)^+]$, 841 $[(2M+H)^+]$.

Beispiel 6

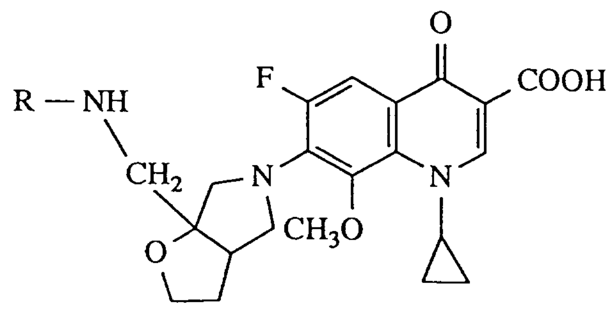

A: R = $(CH_3)_3C$-O-CO
B: R = H x HCl

A: Analog Beispiel 1A setzt man mit 1-Cyclopropyl-6,7-difluor-1,4-dihydro-8-methoxy-4-oxo-3-chinolincarbonsäure um und reinigt das Reaktionsprodukt durch Chromatographie an Kieselgel mit Methylenchlorid/Methanol/17 % wäßriges Ammoniak (470:20:1). Man erhält 7-(1-tert.-Butoxycarbonylaminomethyl-2-oxa-7-aza-bicyclo-[3.3.0]oct-7-yl)-1-cyclopropyl-6,8-difluor-1,4-dihydro-8-methoxy-4-oxo-3-chinolincarbonsäure in 23 %iger Ausbeute. CI-MS: m/e 518 [$(M+H)^+$].

B: 15 mg des Produkts aus der Stufe A werden in einer Mischung aus 1 ml Methanol und 3 Tropfen konzentrierter Salzsäure 1 Tag bei Raumtemperatur stehengelassen. Die Reaktionskontrolle erfolgt dünnschichtchromatographisch (Kieselgel, Methylenchlorid/Methanol/17 % Ammoniak 30:8:1). Nach Anspaltung der Schutzgruppe wird die Lösung eingeengt, der Rückstand mit wenig Ethanol verrieben, abgesaugt und getrocknet.

Ausbeute: 11,8 mg (90 % der Theorie) 7-(1-Aminomethyl-2-oxa-7-aza-bicyclo[3.3.0]oct-7-yl)-1-cyclopropyl-6,8-difluor-1,4-dihydro-8-methoxy-4-oxo-3-chinolincarbonsäure-Hydrochlorid, CI-MS: m/e 418 [-$(M+H)^+$]; daneben ist als Nebenkomponente mit schwächerer Intensität der Methylester nachweisbar: m/e 432.

Beispiel 7

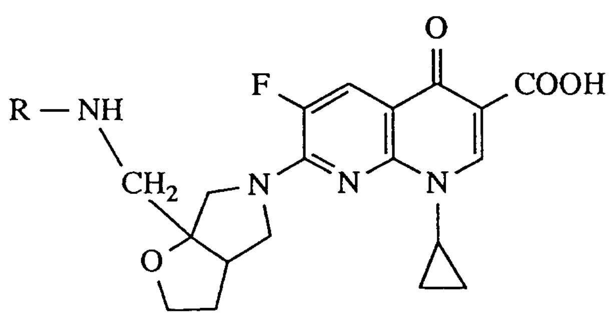

A: R = $(CH_3)_3C$-O-CO
B: R = H x HCl

A: 283 mg (1 mmol) 6-Chlor-1-cyclopropyl-7-fluor-1,4-dihydro-4-oxo-1,8-naphthyridon-3-carbonsäure werden in 5 ml Acetonitril mit 130 mg (1,16 mmol) 1,4-Diazabicyclo[2.2.2]octan und 250 mg (1,03 mmol) 1-tert.-Butoxycarbonylaminomethyl-2-oxa-7-aza-bicyclo[3.3.0]octan versetzt und 2 Stunden bei 50 °C unter Wasserausschluß gerührt. Das ausgefallene Produkt wird abgesaugt, mit Wasser gewaschen, getrocknet und chromatographisch (Kieselgel; Methylenchlorid/Methanol 95:5) gereinigt.

Ausbeute: 87 mg (18 % der Theorie) 7-(1-tert.-Butoxycarbonylaminomethyl-2-oxa-7-aza-bicyclo[3.3.0]oct-7-yl)-1-cyclopropyl-6-fluor-1,4-dihydro-4-oxo-1,8-naphthyridin-3-carbonsäure,
$^1$H-NMR ($CDCl_3$): $\delta$ 8,35 s (1 H), 8,0 ppm d (1 H).

B: 70 mg (0,14 mmol) des Produkts aus Stufe A werden in 1 ml halbkonzentrierter Salzsäure warm gelöst, die Lösung filtriert, eingedampft und der Rückstand mit wenig Ethanol verrührt. Das Salz wird abgesaugt, mit Ethanol gewaschen und getrocknet.
Ausbeute: 45 mg (74 % der Theorie) 7-(1-Aminomethyl-2-oxa-7-aza-bicyclo[3.3.0]oct-7-yl)-1-cyclopropyl-6-fluor-1,4-dihydro-4-oxo-1,8-naphthyridin-3-carbonsäure-Hydrochlorid,
Schmelzpunkt: 220-221 °C (unter Zersetzung).

Beispiel 8

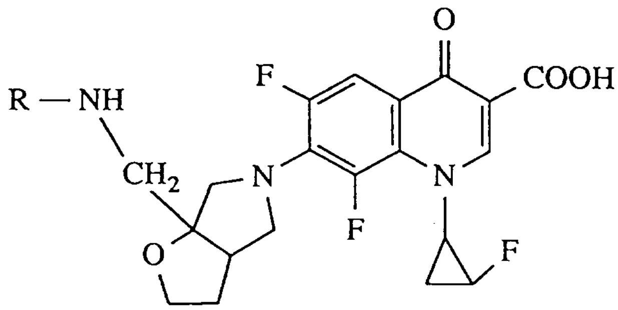

A: R = $(CH_3)_3C$-O-CO
B: R = H x HCl

A: Analog Beispiel 1A setzt man mit 6,7,8-Trifluor-1-(cis-2-fluorcyclopropyl)-1,4-dihydro-4-oxo-3-chinolincarbonsäure zu 7-(1-tert.-Butoxycarbonylaminomethyl-2-oxa-7-aza-bicyclo[3.3.0]oct-7-yl)-6,8-difluor-1-(cis-2-fluorcyclopropyl)-1,4-dihydro-4-oxo-3-chinolincarbonsäure (73 % der Theorie) vom Schmelzpunkt 121-129 °C (unter Zersetzung) um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-6,8-difluor-1-(cis-2-fluorcyclopropyl)-1,4-dihydro-4-oxo-3-chinolincarbonsäure-Hydrochlorid vom Schmelzpunkt 220-227 °C (unter Zersetzung) umgesetzt.

Beispiel 9

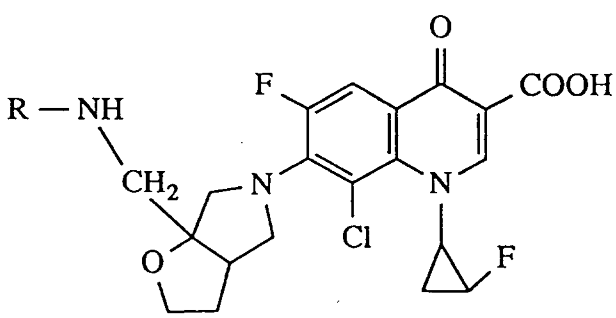

A: R = $(CH_3)_3C$-O-CO
B: R = H x HCl

A: Analog Beispiel 1A setzt man mit 8-Chlor-6,7-difluor-1-(cis-2-fluorcyclopropyl)-1,4-dihydro-4-oxo-3-chinolincarbonsäure zu 7-(1-tert.-Butoxycarbonylaminomethyl-2-oxa-7-aza-bicyclo[3.3.0]oct-7-yl)-8-chlor-6-fluor-1-(cis-2-fluorcyclopropyl)-1,4-dihydro-4-oxo-3-chinolincarbonsäure (93 % der Theorie) vom Schmelzpunkt 133-135 °C (unter Zersetzung) um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-6-fluor-1-(cis-2-fluorcyclopropyl)-1,4-dihydro-4-oxo-3-chinolincarbonsäure-Hydrochlorid vom Schmelzpunkt 180-185 °C (unter Zersetzung) umgesetzt.

Beispiel 10

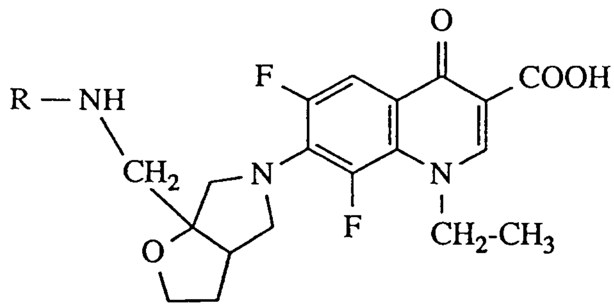

A: R = $(CH_3)_3C$-O-CO
B: R = H x HCl

A: Analog Beispiel 1A setzt man mit 1-Ethyl-6,7,8-trifluor-1,4-dihydro-4-oxo-3-chinolincarborsäure zu 7-(1-tert.-Butoxycarbonylaminomethyl-2-oxa-7-aza-bicyclo[3.3.0]oct-7-yl)-1-ethyl-6,8-difluor-1,4-dihydro-4-oxo-3-chinolincarbonsäure (83 % der Theorie) vom Schmelzpunkt 234-236 ° C (unter Zersetzung) um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-1-ethyl-6,8-difluor-1,4-dihydro-4-oxo-3-chinolincarbonsäure-Hydrochlorid vom Schmelzpunkt 218-223 ° C (unter Zersetzung) umgesetzt.

Beispiel 11

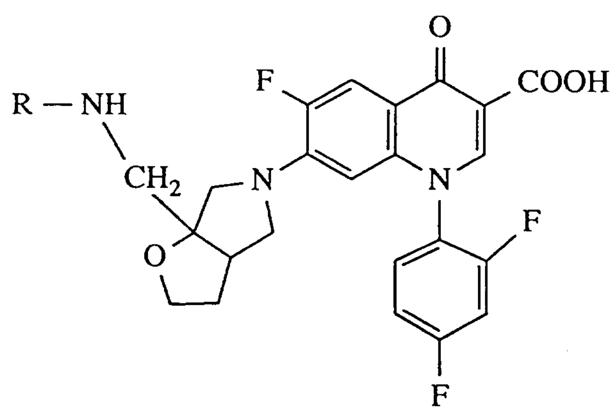

A: R = $(CH_3)_3C$-O-CO
B: R = H x HCl

A: Analog Beispiel 1A setzt man mit 1-(2,4-Difluor-phenyl)-6,7-difluor- 1,4-dihydro-4-oxo-3-chinolincarbonsäure zu 7-(1-tert.-Butoxycarbonylaminomethyl-2-oxa-7-aza-bicyclo[3.3.0]oct-7-yl)-1-(2,4-difluorphenyl)-6-fluor-1,4-dihydro-4-oxo-3-chinolincarbonsäure (93 %) der Theorie) vom Schmelzpunkt 125-130 ° C (unter Zersetzung) um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-1-(2,4-difluorphenyl)-6-fluor-1,4-dihydro-4-oxo-3-chinolincarbonsäure-Hydrochlorid vom Schmelzpunkt 235 (unter Zersetzung) umgesetzt.

Beispiel 12

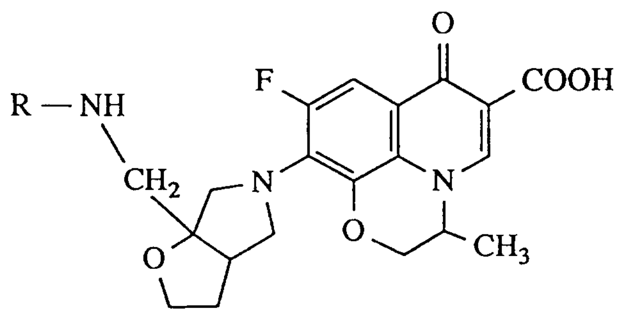

A: R = $(CH_3)_3C$-O-CO
B: R = H x HCl

A: Analog Beispiel 1A setzt man mit 9,10-Difluor-3-methyl-7-oxo-2,3-dihydro-7H-pyrido[1,2,3-de][1,4]-benzoxacin-6-carbonsäure zu 10-(1-tert.-Butoxycarbonylaminomethyl-2-oxa-7-aza-bicyclo[3.3.0]oct-7-yl)-9-fluor-3-methyl-7-oxo-2,3-dihydro-7H-pyrido-[1,2,3-de][1,4]benzoxacin-6-carbonsäure (21 % der Theorie) vom Schmelzpunkt 176-179 °C (unter Zersetzung) um.

B: Analog Beispiel 1B wird das Produkt aus der Stufe A mit halbkonzentrierter Salzsäure zu 10-(1-Aminomethyl-2-oxa-7-aza-bicyclo[3.3.0]oct-7-yl)-9-fluor-3-methyl-7-oxo-2,3-dihydro-7H-pyrido-[1,2,3-de]-[1,4]benzoxacin-6-carbonsäure-Hydrochlorid vom Schmelzpunkt 220-227 °C (unter Zersetzung) umgesetzt.

Beispiel 13

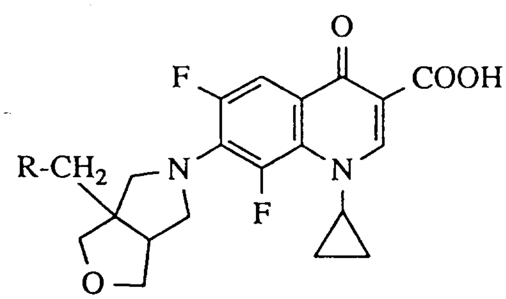

A: R = 3-$NO_2$-$C_6H_4$-CH=N-
B: R = HCl x $NH_2$-

A: 581 mg (4,1 mmol) 1-Aminomethyl-3-oxa-7-azabicyclo[3.3.0]octan werden in 5 ml Acetonitril gelöst und mit einer Lösung von 650 mg (4,3 mmol) 3-Nitrobenzaldehyd in 5 ml Acetonitril versetzt. Man läßt 1 Stunde bei Raumtemperatur stehen, verdünnt mit 3 ml Dimethylformamid, fügt 672 mg (6 mmol) 1,4-Diazabicyclo[2.2.2]octan und 850 mg (3 mmol] 1-Cyclopropyl-6,7,8-trifluor-1,4-dihydro-4-oxo-3-chinolincarbonsäure zu und erhitzt 3 Stunden unter Rückfluß. Der Niederschlag wird abgesaugt, mit Acetonitril und Wasser gewaschen und bei 100 °C im Umluft-Trockenschrank getrocknet.

Ausbeute: 948 mg (60 % der Theorie) 1-Cyclopropyl-6,8-difluor-1,4-dihydro-7-(3-nitrobenzylidenaminomethyl-3-oxa-7-azabicyclo-[3.3.0]oct-7-yl)-4-oxo-3-chinolincarbonsäure,

Schmelzpunkt: 220-230 °C (unter Zersetzung) (aus Dimethylformamid umkristallisiert).

B: 526 mg (1mmol) des Produkts aus Stufe A werden in 12 ml 5 n Salzsäure gelöst. Die Mischung wird mit Dichlormethan extrahiert, die Wasserphase abgetrennt, mit Dichlormethan gewaschen, eingedampft, der Rückstand mit Ethanol verrührt und das erhaltene Salz abgesaugt, mit Ethanol gewaschen und bei

100-120 °C im Hochvakuum getrocknet.
Ausbeute: 370 mg (84 % der Theorie) 7-(1-Aminomethyl-3-oxa-7-azabicyclo[3.3.0]oct-7-yl)-1-cyclopropyl-6,8-difluor-1,4-dihydro-4-oxo-3-chinolincarbonsäure-Hydrochlorid,
Schmelzpunkt: 245-249 °C (unter Zersetzung).

Beispiel 14

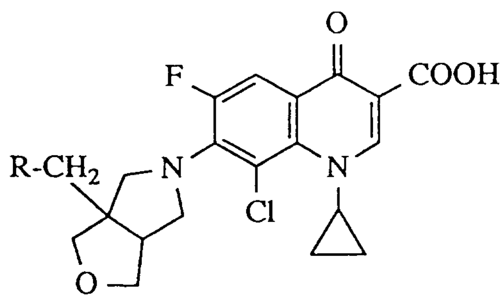

A: R = $3\text{-}NO_2\text{-}C_6H_4\text{-}CH{=}N\text{-}$
B: R = HCl x $NH_2$-

A: Analog Beispiel 13 wird mit 8-Chlor-1-cyclopropyl-6,7-difluor-1,4-dihydro-4-oxo-3-chinolincarbonsäure zu 8-Chlor-1-cyclopropyl-6-fluor-1,4-dihydro-7-(3-nitrobenzylidenaminomethyl)-3-oxa-7-azabicyclo[3.3.0]-oct-7-yl)-4-oxo-3-chinolincarbonsäure umgesetzt.
Schmelzpunkt: 222-224 °C (unter Zersetzung).
B: 910 mg (1,7 mmol) des Produkts aus Beispiel 14 wird in 64 ml Dichlormethan mit 22 ml 3n-Salzsäure 30 Minuten im Ultraschallbad behandelt. Die wäßrige Phase wird abgetrennt, nochmals mit Dichlormethan extahiert, eingeengt und der Rückstand mit Ethanol verrührt. Der Niederschlag wird abgesaugt und bei 90 °C im Hochvakuum getrocknet.
Ausbeute: 700 mg (91 % der Theorie) 7-(1-Aminomethyl-3-oxa-7-azabicyclo[3.3.0]oct-7-yl)-8-chlor-1-cyclopropyl-6-fluor-1,4 -dihydro-4-oxo-3-chinolincarbonsäure-Hydrochlorid,
Schmelzpunkt: sintert ab 168 °C, bei 188 °C Zersetzung unter Aufschäumen.

**Patentansprüche**

**1.** Verbindungen der Formel (I)

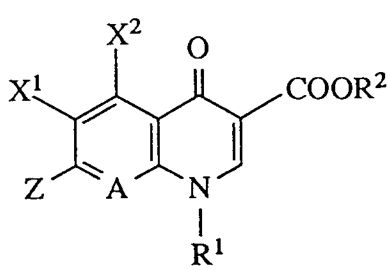

(I),

in welcher
$X^1$ für Halogen oder Nitro,
$X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Methyl,
$R^1$ für Alkyl mit 1 bis 4 Kohlenstoffatomen, Alkenyl mit 2 bis 4 Kohlenstoffatomen, gegebenenfalls durch Halogen substituiertes Cycloalkyl mit 3 bis 6 Kohlenstoffatomen, Bicyclo[1.1.1]-

pent-1-yl, 1,1-Dimethylpropargyl, 3-Oxetanyl, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl,

Z für einen Rest der Struktur

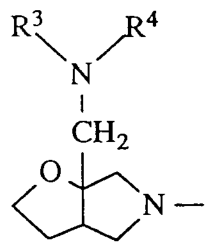 , 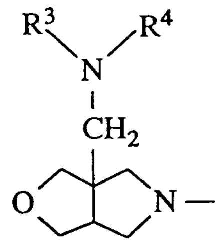 oder 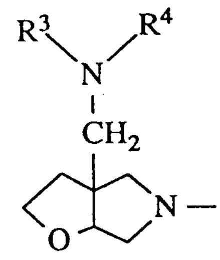

steht, worin

$R^3$ für Wasserstoff, Methyl oder Ethyl,

$R^4$ für Wasserstoff, Acyl mit 1 bis 3 Kohlenstoffatomen, Alkoxycarbonyl mit 1 bis 4 Kohlenstoffatomen im Alkylteil oder Methyl steht und

A für N oder C-$R^5$ steht, worin

$R^5$ für Wasserstoff, Halogen, Methyl, Alkenyl mit 2 bis 3 Kohlenstoffatomen, Alkinyl mit 2 bis 3 Kohlenstoffatomen, Hydroxy oder Methoxy steht oder auch gemeinsam mit $R^1$ eine Brücke der Struktur

$$-O-CH_2-{}^*\underset{|}{C}H-CH_3, \quad -S-CH_2-{}^*\underset{|}{C}H-CH_3 \quad \text{oder} \quad -CH_2-CH_2-{}^*\underset{|}{C}H-CH_3$$

$$-O-CH_2-\underset{|}{N}-R^6,$$

worin $R^6$ Wasserstoff, Methyl oder Formyl bedeutet, bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

**2.** Verbindungen der Formel (I) gemäß Anspruch 1, in welcher

$X^1$ für Fluor,

$X^2$ für Wasserstoff, Amino, Methylamino, Hydroxy, Methoxy, Fluor, Chlor, Brom oder Methyl,

$R^1$ für Alkyl mit 1 bis 3 Kohlenstoffatomen, Vinyl, Cycloalkyl mit 3 bis 4 Kohlenstoffatomen, 2-(cis- oder trans-)Fluorcyclopropyl, gegebenenfalls durch 1 oder 2 Fluoratome substituiertes Phenyl,

$R^2$ für Wasserstoff, gegebenenfalls durch Amino, Methylamino oder Dimethylamino substituiertes Alkyl mit 1 bis 2 Kohlenstoffatomen oder (5-Methyl-2-oxo-1,3-dioxol-4-yl)-methyl,

Z für einen Rest der Struktur

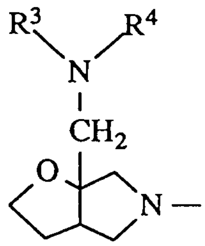 , 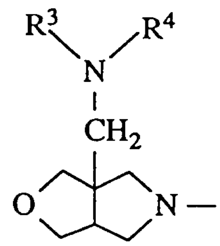 oder 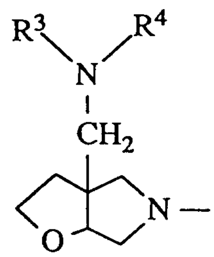

steht, worin

- $R^3$ für Wasserstoff oder Methyl,
- $R^4$ für Wasserstoff, Acyl mit 1 bis 2 Kohlenstoffatomen, Alkoxycarbonyl mit 1 bis 4 Kohlenstoffatomen im Alkylteil oder Methyl steht und
- A für N oder C-$R^5$ steht, worin
- $R^5$ für Wasserstoff, Fluor, Chlor, Methyl, Vinyl, Ethinyl oder Methoxy steht oder auch gemeinsam mit $R^1$ eine Brücke der Struktur

$$-O-CH_2-{}^{*}\underset{|}{C}H-CH_3 \quad \text{oder} \quad -O-CH_2-\underset{|}{N}-CH_3$$

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

**3.** Verbindungen der Formel (I) gemäß Anspruch 1, in welcher

- $X^1$ für Fluor,
- $X^2$ für Wasserstoff, Amino oder Fluor,
- $R^1$ für Alkyl mit 1 bis 2 Kohlenstoffatomen, Cyclopropyl, 2-(cis- oder trans-)Fluorcyclopropyl, gegebenenfals durch 1 oder 2 Fluoratome substituiertes Phenyl,
- $R^2$ für Wasserstoff oder Alkyl mit 1 bis 2 Kohlenstoffatomen,
- Z für einen Rest der Struktur

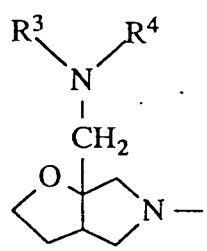 , 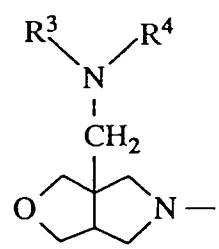 oder 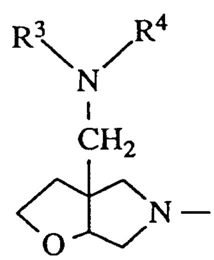

steht, worin

- $R^3$ für Wasserstoff,
- $R^4$ für Wasserstoff oder Alkoxcarbonyl mit 1 bis 4 Kohlenstoffatomen im Alkylteil steht und
- A für N oder C-$R^5$ steht, worin
- $R^5$ für Wasserstoff, Fluor, Chlor oder Methoxy steht oder auch gemeinsam mit $R^1$ eine Brücke der Struktur

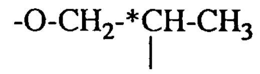

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

**4.** Verfahren zur Herstellung von Verbindungen der Formel (I) gemäß Ansprüchen 1 bis 3, dadurch gekennzeichnet, daß Verbindungen der Formel (II)

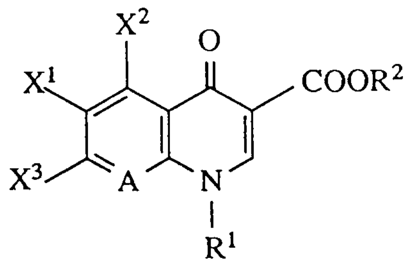

(II),

in welcher

A, $R^1$, $R^2$, $X^1$ und $X^2$ die oben angegebene Bedeutung haben, und

$X^3$ für Halogen, insbesondere für Fluor oder Chlor, steht,

mit Verbindungen der Formel (III)

Z'-H (III),

in welcher

Z' die oben für Z angegebene Bedeutung hat oder für einen Rest Z steht, dessen Aminogruppe gegebenenfalls durch eine Schutzgruppe blockiert sein kann,

gegebenenfalls in Gegenwart von Säurefängern umgesetzt und gegebenenfalls vorhandene Schutzgruppen abgespalten werden.

**5.** Verbindungen der allgemeinen Formel (IIIa)

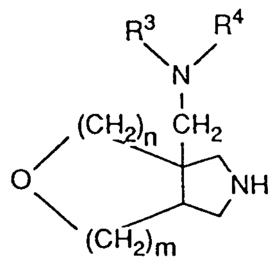

(IIIa),

worin,

n und m 0,1 oder 2 sein können, die Summe n + m jedoch immer 2 ist,

$R^3$ für Wasserstoff, Methyl oder Ethyl,

$R^4$ für Wasserstoff, Acyl mit 1 bis 3 Kohlenstoffatomen, Alkoxycarbonyl mit 1 bis 4 Kohlenstoffatomen im Alkylteil oder Methyl steht,

in Form ihrer Racemate, Enantiomeren oder Diastereomeren.

**6.** Verbindungen gemäß Anspruch 5 der Formel (IIIa) aus der Gruppe bestehend aus

1-Aminomethyl-2-oxa-7-aza-bicyclo[3.3.0]octan,

1-Methylaminomethyl-2-oxa-7-aza-bicyclo[3.3.0]octan,

1-Dimethylaminomethyl-2-oxa-7-aza-bicyclo[3.3.0]octan,

1-Ethylaminomethyl-2-oxa-7-aza-bicyclo[3.3.0]octan,
1-Formylaminomethyl-2-oxa-7-aza-bicyclo[3.3.0]octan,
1-Acetylaminomethyl-2-oxa-7-aza-bicyclo[3.3.0]octan,
1-Methoxycarbonylaminomethyl-2-oxa-7-aza-bicyclo[3.3.0]octan,
1-Ethoxycarbonylaminomethyl-2-oxa-7-aza-bicyclo[3.3.0]octan,
1-tert.-Butoxycarbonylaminomethyl-2-oxa-7-aza-bicyclo[3.3.0]octan,
1-Aminomethyl-3-oxa-7-aza-bicyclo[3.3.0]octan,
1-Methylaminomethyl-3-oxa-7-aza-bicyclo[3.3.0]octan,
1-Dimethylaminomethyl-3-oxa-7-aza-bicyclo[3.3.0]octan,
1-Ethylaminomethyl-3-oxa-7-aza-bicyclo[3.3.0]octan,
1-Formylaminomethyl-3-oxa-7-aza-bicyclo[3.3.0]octan,
1-Acetylaminomethyl-3-oxa-7-aza-bicyclo[3.3.0]octan,
1-Methoxycarbonylaminomethyl-3-oxa-7-aza-bicyclo[3.3.0]octan,
1-Ethoxycarbonylaminomethyl-3-oxa-7-aza-bicyclo[3.3.0]octan,
1-tert.-Butoxycarbonylaminomethyl-3-oxa-7-aza-bicyclo[3.3.0]octan,
5-Aminomethyl-2-oxa-7-aza-bicyclo[3.3.0]octan,
5-Methylaminomethyl-2-oxa-7-aza-bicyclo[3.3.0]octan,
5-Dimethylaminomethyl-2-oxa-7-aza-bicyclo[3.3.0]octan,
5-Ethylaminomethyl-2-oxa-7-aza-bicyclo[3.3.0]octan,
5-Formylaminomethyl-2-oxa-7-aza-bicyclo[3.3.0]octan,
5-Acetylaminomethyl-2-oxa-7-aza-bicyclo[3.3.0]octan,
5-Acetylaminomethyl-2-oxa-7-aza-bicyclo[3.3.0]octan,
5-Methoxycarbonylaminomethyl-2-oxa-7-aza-bicyclo[3.3.0]octan,
5-Ethoxycarbonylaminomethyl-2-oxa-7-aza-bicyclo[3.3.0]octan,
5-tert.-Butoxycarbonylaminomethyl-2-oxa-7-aza-bicyclo[3.3.0]octan,
sowie deren Racemate als auch deren enantiomeren- oder diastereomerenreine Verbindungen.

**7.** Verbindungen gemäß Ansprüchen 1 bis 3 zur Bekämpfung von Krankheiten.

**8.** Verbindungen gemäß Ansprüchen 1 bis 3 zur Bekämpfung von bakteriellen Infektionen.

**9.** Arzneimittel enthalten Verbindungen gemäß Ansprüchen 1 bis 3.

**10.** Antibakterielle Mittel enthaltend Verbindungen gemäß Ansprüchen 1 bis 3.

## Claims

**1.** Compounds of the formula (I)

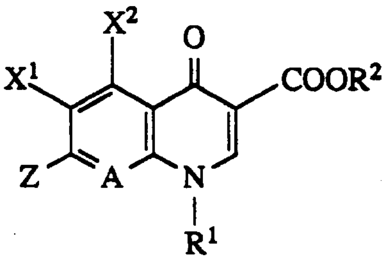

(I),

in which

$X^1$ represents halogen or nitro,

$X^2$ represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen or methyl,

$R^1$ represents alkyl having 1 to 4 carbon atoms, alkenyl having 2 to 4 carbon atoms, cycloalkyl having 3 to 6 carbon atoms, which is optionally substituted by halogen, bicyclo[1.1.1]pent-1-yl, 1,1-dimethylpropargyl, 3-oxetanyl, 2-hydroxyethyl, 2-fluoroethyl, methoxy, amino, methylamino, ethylamino, dimethylamino, or phenyl which is optionally substituted by 1 or 2

fluorine atoms,

$R^2$ represents hydrogen, alkyl having 1 to 4 carbon atoms, which is optionally substituted by hydroxyl, methoxy, amino, methylamino or dimethylamino, or (5-methyl-2-oxo-1,3-dioxol-4-yl)-methyl,

Z represents a residue of the structure

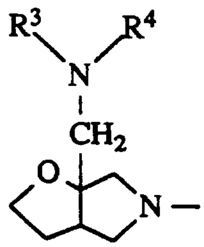 , 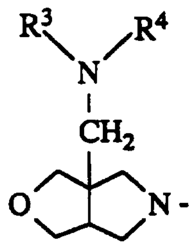 or 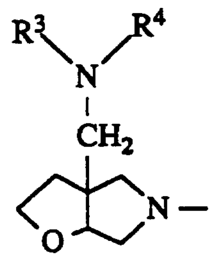

in which

$R^3$ represents hydrogen, methyl or ethyl,

$R^4$ represents hydrogen, acyl having 1 to 3 carbon atoms, alkoxycarbonyl having 1 to 4 carbon atoms in the alkyl moiety, or methyl, and

A represents N or C-$R^5$, in which

$R^5$ represents hydrogen, halogen, methyl, alkenyl having 2 to 3 carbon atoms, alkinyl having 2 to 3 carbon atoms, hydroxyl or methoxy, or, together with $R^1$, can form a bridge of the structure

$$-O-CH_2-{}^*CH-CH_3,\ -S-CH_2-{}^*CH-CH_3 \text{ or } -CH_2-CH_2-{}^*CH-CH_3$$

$$-O-CH_2-N-R^6,$$

in which $R^6$ denotes hydrogen, methyl or formyl,

and their pharmaceutically utilisable hydrates and acid-addition salts, as well as the alkali metal, alkaline earth metal, silver and guanidinium salts of the underlying carboxylic acids.

2. Compounds of the formula (I) according to Claim 1, in which

$X^1$ represents fluorine,

$X^2$ represents hydrogen, amino, methylamino, hydroxyl, methoxy, fluorine, chlorine, bromine or methyl,

$R^1$ represents alkyl having 1 to 3 carbon atoms, vinyl, cycloalkyl having 3 to 4 carbon atoms, 2-(cis- or trans-)fluorocyclopropyl, or phenyl which is optionally substituted by 1 or 2 fluorine atoms,

$R^2$ represents hydrogen, alkyl having 1 to 2 carbon atoms, which is optionally substituted by amino, methylamino or dimethylamino, or (5-methyl-2-oxo-1,3-dioxol-4-yl)-methyl,

Z represents a residue of the structure

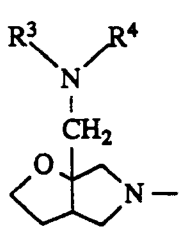 , 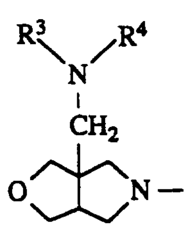 or 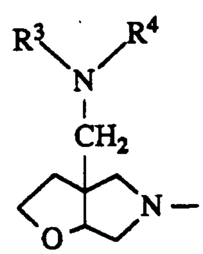

in which

$R^3$ represents hydrogen or methyl,

$R^4$ represents hydrogen, acyl having 1 to 2 carbon atoms, alkoxycarbonyl having 1 to 4 carbon atoms in the alkyl moiety, or methyl, and

A represents N or C-$R^5$, in which

$R^5$ represents hydrogen, fluorine, chlorine, methyl, vinyl, ethinyl, or methoxy, or, together with $R^1$, can form a bridge of the structure

$$-O-CH_2-{}^*CH-CH_3 \quad \text{or} \quad -O-CH_2-N-CH_3 \;,$$

and their pharmaceutically utilisable hydrates and acid addition salts, as well as the alkali metal, alkaline earth metal, silver and guanidinium salts of the underlying carboxylic acids.

**3.** Compounds of the formula (I) according to Claim 1, in which

$X^1$ represents fluorine,

$X^2$ represents hydrogen, amino or fluorine,

$R^1$ represents alkyl having 1 to 2 carbon atoms, cyclopropyl, 2-(cis- or trans-)fluorocyclopropyl, or phenyl which is optionally substituted by 1 or 2 fluorine atoms,

$R^2$ represents hydrogen or alkyl having 1 to 2 carbon atoms,

Z represents a residue of the structure

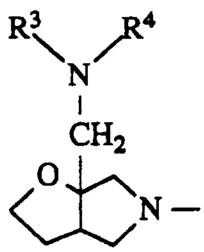 , 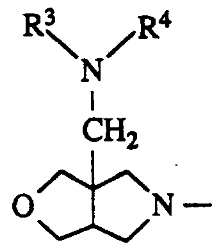 or 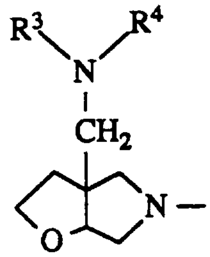

in which

$R^3$ represents hydrogen,

$R^4$ represents hydrogen or alkoxycarbonyl having 1 to 4 carbon atoms in the alkyl moiety and

A represents N or C-$R^5$, in which

$R^5$ represents hydrogen, fluorine, chlorine or methoxy, or, together with $R^1$, can form a bridge of the structure

$$-O-CH_2-{}^*CH-CH_3$$

and their pharmaceutically utilisable hydrates and acid addition salts, as well as the alkali metal, alkaline earth metal, silver and guanidinium salts of the underlying carboxylic acids.

**4.** Process for preparing compounds of the formula (I) according to Claims 1 to 3, characterised in that compounds of the formula (II)

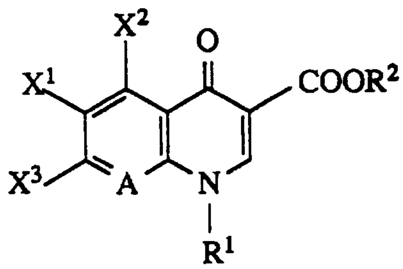

(II), (II)

in which

A, $R^1$, $R^2$, $X^1$ and $X^2$ have the abovementioned meaning, and

$X^3$ represents halogen, in particular fluorine or chlorine,

are reacted with compounds of the formula (III)

Z'-H (III),

in which

Z' has the meaning given above for Z or represents a residue Z whose amino group can optionally be blocked by a protective group,

optionally in the presence of acid-capturing agents, and protective groups which may optionally be present are eliminated.

**5.** Compounds of the general formula (IIIa)

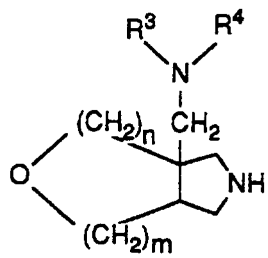

(IIIa),

in which

n and m can be 0, 1 or 2, but the sum n + m is always 2,

$R^3$ represents hydrogen, methyl or ethyl,

$R^4$ represents hydrogen, acyl having 1 to 3 carbon atoms, alkoxycarbonyl having 1 to 4 carbon atoms in the alkyl moiety, or methyl,

in the form of their racemates, enantiomers or diastereomers.

**6.** Compounds according to Claim 5 of the formula (IIIa) from the series comprising

1-Aminomethyl-2-oxa-7-aza-bicyclo[3.3.0]octane,
1-Methylaminomethyl-2-oxa-7-aza-bicyclo[3.3.0]octane,
1-Dimethylaminomethyl-2-oxa-7-aza-bicyclo[3.3.0]octane,
1-Ethylaminomethyl-2-oxa-7-aza-bicyclo[3.3.0]octane,
1-Formylaminomethyl-2-oxa-7-aza-bicyclo[3.3.0]octane,
1-Acetylaminomethyl-2-oxa-7-aza-bicyclo[3.3.0]octane,
1-Methoxycarbonylaminomethyl-2-oxa-7-aza-bicyclo[3.3.0]octane,
1-Ethoxycarbonylaminomethyl-2-oxa-7-aza-bicyclo[3.3.0]octane,
1-tert-Butoxycarbonylaminomethyl-2-oxa-7-aza-bicyclo[3.3.0]octane,
1-Aminomethyl-3-oxa-7-aza-bicyclo[3.3.0]octane,
1-Methylaminomethyl-3-oxa-7-aza-bicyclo[3.3.0]octane,
1-Dimethylaminomethyl-3-oxa-7-aza-bicyclo[3.3.0]octane,
1-Ethylaminomethyl-3-oxa-7-aza-bicyclo[3.3.0]octane,
1-Formylaminomethyl-3-oxa-7-aza-bicyclo[3.3.0]octane,

1-Acetylaminomethyl-3-oxa-7-aza-bicyclo[3.3.0]octane,
1-Methoxycarbonylaminomethyl-3-oxa-7-aza-bicyclo[3.3.0]octane,
1-Ethoxycarbonylaminomethyl-3-oxa-7-aza-bicyclo[3.3.0]octane,
1-tert-Butoxycarbonylaminomethyl-3-oxa-7-aza-bicyclo[3.3.0]octane,
5-Aminomethyl-2-oxa-7-aza-bicyclo[3.3.0]octane,
5-Methylaminomethyl-2-oxa-7-aza-bicyclo[3.3.0]octane,
5-Dimethylaminomethyl-2-oxa-7-aza-bicyclo[3.3.0]octane,
5-Ethylaminomethyl-2-oxa-7-aza-bicyclo[3.3.0]octane,
5-Formylaminomethyl-2-oxa-7-aza-bicyclo[3.3.0]octane,
5-Acetylaminomethyl-2-oxa-7-aza-bicyclo[3.3.0]octane,
5-Acetylaminomethyl-2-oxa-7-aza-bicyclo[3.3.0]octane,
5-Methoxycarbonylaminomethyl-2-oxa-7-aza-bicyclo[3.3.0]octane,
5-Ethoxycarbonylaminomethyl-2-oxa-7-aza-bicyclo[3.3.0]octane,
5-tert-Butoxycarbonylaminomethyl-2-oxa-7-aza-bicyclo[3.3.0]octane,
as well as their racemates and their enantiomerically or diastereomerically pure compounds.

**7.** Compounds according to Claims 1 to 3 for controlling diseases.

**8.** Compounds according to Claims 1 to 3 for controlling bacterial infections.

**9.** Medicaments containing compounds according to Claims 1 to 3.

**10.** Antibacterial agents containing compounds according to Claims 1 to 3.

## Revendications

**1.** Composés de formule (I)

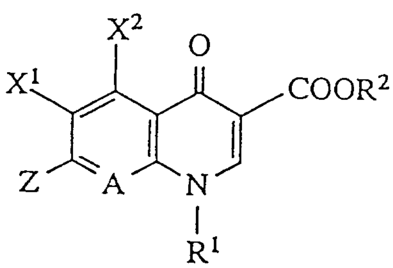

(I),

dans laquelle

$X^1$ représente un halogène ou un groupe nitro,

$X^2$ représente de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, halogéno ou méthyle,

$R^1$ est un groupe alkyle ayant 1 à 4 atomes de carbone, alcényle ayant 2 à 4 atomes de carbone, cycloalkyle de 3 à 6 atomes de carbone éventuellement substitué par un halogène, bicyclo[1.1.1]pent-1-yle, 1,1-diméthylpropargyle, 3-oxétanyle, 2-hydroxyéthyle, 2-fluoréthyle, méthoxy, amino, méthylamino, éthylamino, diméthylamino, phényle éventuellement substitué par 1 ou 2 atomes de fluor,

$R^2$ est de l'hydrogène, un groupe alkyle ayant 1 à 4 atomes de carbone éventuellement substitué par un radical hydroxy, méthoxy, amino, méthylamino ou diméthylamino ou un groupe (5-méthyl-2-oxo-1,3-dioxol-4-yl)-méthyle,

Z est un reste de structure

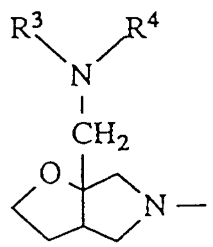 , 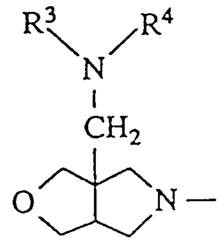 ou 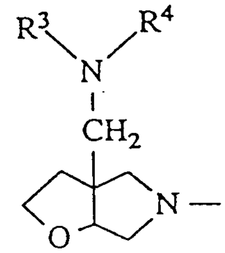

où

$R^3$ représente de l'hydrogène, un groupe méthyle ou éthyle,

$R^4$ est de l'hydrogène, un groupe acyle ayant 1 à 3 atomes de carbone, alkoxycarbonyle ayant 1 à 4 atomes de carbone dans la partie alkyle, ou un groupe méthyle et

A représente N ou un groupe $C-R^5$ dans lequel

$R^5$ est de l'hydrogène, un halogène, un groupe méthyle, alcényle ayant 2 ou 3 atomes de carbone, alcynyle ayant 2 ou 3 atomes de carbone, hydroxy ou méthoxy ou peut aussi former conjointement avec $R^1$ un pont de structure

$$-O-CH_2-{}^{*}\underset{|}{C}H-CH_3, \quad -S-CH_2-{}^{*}\underset{|}{C}H-CH_3 \quad \text{ou} \quad -CH_2-CH_2-{}^{*}\underset{|}{C}H-CH_3$$

$$-O-CH_2-\underset{|}{N}-R^6,$$

où $R^6$ est de l'hydrogène, un groupe méthyle ou formyle,

et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

**2.** Composés de formule (I) suivant la revendication 1, dans laquelle

$X^1$ représente le fluor,

$X^2$ est l'hydrogène, un groupe amino, méthylamino, hydroxy, méthoxy, le fluor, le chlore, le brome ou un groupe méthyle,

$R^1$ est un groupe alkyle ayant 1 à 3 atomes de carbone, vinyle, cycloalkyle ayant 3 à 4 atomes de carbone, 2-(cis- ou trans)fluorocyclopropyle, phényle éventuellement substitué par 1 ou 2 atomes de fluor,

$R^2$ est de l'hydrogène, un groupe alkyle ayant 1 ou 2 atomes de carbone éventuellement substitué par un radical amino, méthylamino ou diméthylamino, ou un groupe (5-méthyl-2-oxo-1,3-dioxol-4-yl)-méthyle,

Z représente un reste de structure

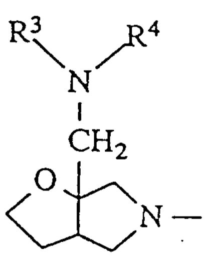 , 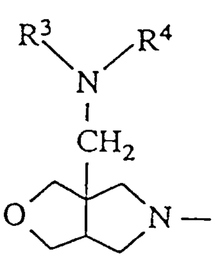 ou 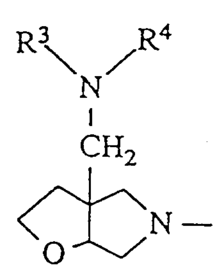

où

$R^3$ est de l'hydrogène ou un groupe méthyle,

$R^4$ est de l'hydrogène, un groupe acyle ayant 1 ou 2 atomes de cabone, alkoxycarbonyle ayant

1 à 4 atomes de carbone dans la partie alkyle, ou un groupe méthyle et

A représente N ou un groupe C-$R^5$, dans lequel

$R^5$ est de l'hydrogène, du fluor, du chlore, un groupe méthyle, vinyle, éthynyle ou méthoxy ou peut aussi former conjointement avec $R^1$ un pont de structure

$$-O-CH_2-{}^{*}CH-CH_3 \quad \text{ou} \quad -O-CH_2-N-CH_3$$

et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

**3.** Composés de formule (I) suivant la revendication 1, dans laquelle

$X^1$ représente du fluor,

$X^2$ est de l'hydrogène, un groupe amino ou du fluor,

$R^1$ est un groupe alkyle ayant 1 à 2 atomes de carbone, cyclopropyle, 2-(cis- ou trans)-fluorocyclopropyle, phényle éventuellement substitué par 1 ou 2 atomes de fluor,

$R^2$ est de l'hydrogène ou un groupe alkyle ayant 1 ou 2 atomes de carbone,

Z représente un reste de structure

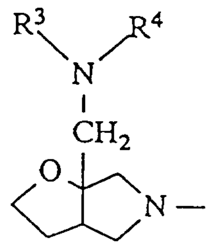 , 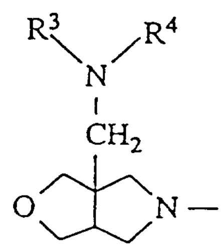 ou 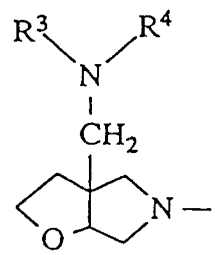

où

$R^3$ est de l'hydrogène,

$R^4$ est de l'hydrogène ou un groupe alkoxycarbonyle ayant 1 à 4 atomes de carbone dans la partie alkyle et

A représente N ou un groupe C-$R^5$, dans lequel

$R^5$ est de l'hydrogène, du fluor, du chlore ou un groupe méthoxy ou peut aussi former conjointement avec $R^1$ un pont de structure

$$-O-CH_2-{}^{*}CH-CH_3$$

et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

**4.** Procédé de production de composés de formule (I) suivant les revendications 1 à 3, caractérisé en ce qu'on fait réagir des composés de formule (II)

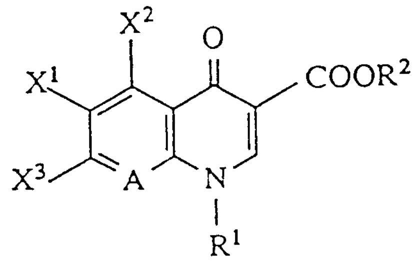

(II),

dans laquelle

A, $R^1$, $R^2$, $X^1$ et $X^2$ ont les définitions indiquées ci-dessus

et

$X^3$ représente un halogène, en particulier le fluor ou le chlore,

avec des composés de formule (III)

Z'-H (III)

dans laquelle

Z' a la définition indiquée ci-dessus pour Z ou représente un reste Z dont le groupe amino peut être protégé, le cas échéant, par un groupe protecteur,

le cas échéant en présence d'accepteurs d'acides, et on élimine les groupes protecteurs éventuellement présents.

**5.** Composés de formule générale (IIIa)

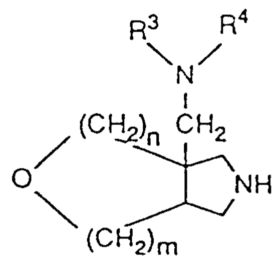

(IIIa),

dans laquelle

n et m peuvent avoir la valeur 0, 1 ou 2, la somme n + m étant cependant toujours égale à 2,

$R^3$ est de l'hydrogène, un groupe méthyle ou éthyle,

$R^4$ est de l'hydrogène, un groupe acyle ayant 1 à 3 atomes de carbone, alkoxycarbonyle ayant 1 à 4 atomes de carbone dans la partie alkyle, ou un groupe méthyle,

sous forme de leurs racémates, énantiomères ou diastéréoisomères.

**6.** Composés suivant la revendication 5 de formule (IIIa), du groupe consistant en :

1-aminométhyl-2-oxa-7-aza-bicyclo[3.3.0]octane,
1-méthylaminométhyl-2-oxa-7-aza-bicyclo[3.3.0]octane,
1-diméthylaminométhyl-2-oxa-7-aza-bicyclo[3.3.0]octane,
1-éthylaminométhyl-2-oxa-7-aza-bicyclo[3.3.0]octane,
1-formylaminométhyl-2-oxa-7-aza-bicyclo[3.3.0]octane,
1-acétylaminométhyl-2-oxa-7-aza-bicyclo[3.3.0]octane,
1-méthoxycarbonylaminométhyl-2-oxa-7-aza-bicyclo[3.3.0]octane,
1-éthoxycarbonylaminométhyl-2-oxa-7-aza-bicyclo[3.3.0]octane,
1-tertio-butoxycarbonylaminométhyl-2-oxa-7-aza-bicyclo[3.3.0]octane,
1-aminométhyl-3-oxa-7-aza-bicyclo[3.3.0]octane,
1-méthylaminométhyl-3-oxa-7-aza-bicyclo[3.3.0]octane,
1-diméthylaminométhyl-3-oxa-7-aza-bicyclo[3.3.0]octane,
1-éthylaminométhyl-3-oxa-7-aza-bicyclo[3.3.0]octane,
1-formylaminométhyl-3-oxa-7-aza-bicyclo[3.3.0]octane,

1-acétylaminométhyl-3-oxa-7-aza-bicyclo[3.3.0]octane,
1-méthoxycarbonylaminométhyl-3-oxa-7-aza-bicyclo[3.3.0]octane,
1-éthoxycarbonylaminométhyl-3-oxa-7-aza-bicyclo[3.3.0]octane,
1-tertio-butoxycarbonylaminométhyl-3-oxa-7-aza-bicyclo[3.3.0]octane,
5-aminométhyl-2-oxa-7-aza-bicyclo[3.3.0]octane,
5-méthylaminométhyl-2-oxa-7-aza-bicyclo[3.3.0]octane,
5-diméthylaminométhyl-2-oxa-7-aza-bicyclo[3.3.0]octane,
5-éthylaminométhyl-2-oxa-7-aza-bicyclo[3.3.0]octane,
5-formylaminométhyl-2-oxa-7-aza-bicyclo[3.3.0]octane,
5-acétylaminométhyl-2-oxa-7-aza-bicyclo[3.3.0]octane,
5-acétylaminométhyl-2-oxa-7-aza-bicyclo[3.3.0]octane,
5-méthoxycarbonylaminométhyl-2-oxa-7-aza-bicyclo[3.3.0]octane,
5-éthoxycarbonylaminométhyl-2-oxa-7-aza-bicyclo[3.3.0]octane,
5-tertio-butoxycarbonylaminométhyl-2-oxa-7-aza-bicyclo[3.3.0]octane,

ainsi que leurs racémates de même que leurs composés énantiomères ou diastéréo-isomères à l'état pur.

**7.** Composés suivant les revendications 1 à 3, destinés à combattre des maladies.

**8.** Composés suivant les revendications 1 à 3, destinés à combattre des infections bactériennes.

**9.** Médicaments contenant des composés suivant les revendications 1 à 3.

**10.** Compositions antibactériennes contenant des composés suivant les revendications 1 à 3.